# EUROPEAN PATENT SPECIFICATION

(11) **EP 0 755 916 B1**
(45) Date of publication and mention of the grant of the patent: **25.04.2001**
(21) Application number: 96111678.7
(22) Date of filing: 19.07.1996
(51) Int. Cl.: C07C 227/00, C07C 227/06, G03G 5/06

(54) **Process for the preparation of amine compound**
Verfahren zur Herstellung einer Aminoverbindung
Procédé pour la préparation de composé aminé

(30) Priority: 21.07.1995 JP 20683195
(43) Date of publication of application: 29.01.1997
(73) Proprietor: FUJI XEROX CO., LTD., Minato-ku, Tokyo (JP)
(72) Inventor: Nukada, Katsumi, Minami Ashigara-shi, Kanagawa (JP)
(74) Representative: Boeters, Hans Dietrich, Dr.

(56) References cited:
- EP-A- 0 713 149
- US-A- 3 180 730
- US-A- 5 080 989
- DATABASE WPI Section Ch, Week 9128 Derwent Publications Ltd., London, GB; Class E14, AN 91-203651 XP002043097 & JP 03 127 765 A (RICOH KK) , 30 May 1991
- PATENT ABSTRACTS OF JAPAN vol. 017, no. 416 (P-1584), 3 August 1993 & JP 05 080550 A (KAO CORP), 2 April 1993,
- PATENT ABSTRACTS OF JAPAN vol. 012, no. 361 (P-763), 28 September 1988 & JP 63 113462 A (FUJI XEROX CO LTD), 18 May 1988,

## Description

### FIELD OF THE INVENTION

The present invention relates to a novel process for the preparation of an amine compound useful as an electric charge-transporting material or a starting material of electric charge-transporting polymer.

### BACKGROUND OF THE INVENTION

As amine compounds, compounds represented by formulae (II), (III) and (IV) for use in the present invention not only can be mixed with a resin to form an electric charge-transporting layer but also can be used as a starting material of electric charge-transporting polymer as described in JP-A-63-113462 (The term "JP-A" as used herein means an "unexamined published Japanese patent application") and JP-A-5-80550.

These electric charge-transporting materials and electric charge-transporting polymers must meet various requirements for solubility, mobility and matching of oxidation potential (ionization potential). In order to meet these requirements, it has been commonly practiced to introduce substituents into chemical substances which have been heretofore used to control the physical properties thereof. The ionization potential of these electric charge-transporting polymers is mostly determined by the electric charge-transporting monomers incorporated therein. Therefore, it is important to make the ionization potential of the electric charge-transporting monomers controllable.

To date, as a process for the preparation of an electric charge-transporting material having an alkylenecarboxylic acid ester group there has been proposed in JP-A-5-80550 a process which comprises introducing a chloromethyl group into the reaction system, reacting the reaction system with Mg to form a Grignard reagent, and then reacting the Grignard reagent with carbon dioxide so that it is converted to a carboxylic acid which is then esterified.

However, the foregoing process for the preparation of an electric charge-transporting material is disadvantageous in that the chloromethyl group used is very reactive and thus cannot be introduced into the starting material in the initial stage of the reaction. Therefore, it is necessary in this preparation process that a skeleton such as triarylamine and tetraarylbenzidine be previously formed, followed by the conversion of a methyl group introduced into the starting material in the initial stage into a chloromethyl group. Alternatively, it is necessary that an unsubstituted starting material be used to form a skeleton such as triarylamine and tetraarylbenzidine, followed by a direct chloromethylation. Alternatively, it is necessary that a starting material into which a formyl group has been introduced be reduced to a hydroxyl group which is then converted with thionyl chloride or the like to a chloromethyl group.

However, since an electric charge-transporting material having a skeleton such as triarylamine and tetraarylbenzidine is very reactive, it can easily cause a reaction involving a direct chlorine substitution on an aromatic ring during chlorination reaction, making it substantially impossible to selectively convert the methyl group which has been previously introduced to chloromethyl group. Further, the process which comprises the use of an unsubstituted starting material, followed by a direct chloromethylation, is disadvantageous in that the chloromethyl group is introduced into only the position para to the nitrogen atom which is to be substituted, restricting the control over the ionization potential. Moreover, the process which comprises introducing a formyl group into the starting material, followed by the conversion to chloromethyl group, is disadvantageous in that it requires a prolonged reaction procedure.

On the other hand, a coupling free from divalent hydrocarbon group T, i.e., coupling of a halogenated benzoic acid with aniline is reported in Org. Syn. Coll. Vol. 2, 15 (1943). Thus, it is known that this coupling can substitute chlorine atom which can hardly be substituted. However, this substitution is specific for ortho position. Further, it is described in J. Am. Chem. Soc., 62, 3208 (1940) that p-iodobenzoic acid cannot undergo coupling reaction with diphenylamine unless it is used in the form of methylesterification product. These facts show that carboxylic acid group has a very great effect on the coupling reaction and the feasibility of the coupling reaction cannot be judged only the similarity of the reaction. Accordingly, the synthesis method of the present invention has never been disclosed.

### SUMMARY OF THE INVENTION

An object of the present invention is to provide a preparation process which allows easy synthesis of amine compounds represented by formulae (II), (III) and (IV) described below useful as electric charge-transporting material and starting material of electric charge-transporting polymer.

The inventor made extensive studies of a process for the preparation of amine compounds represented by formulae (II), (III) and (IV). As a result, it was found that the use of a compound represented by formula (I) as a starting material makes it possible to easily change the position of substituents as well as control the ionization potential. It was also found that the use of the compound (I) provides a highly stable intermediate that leads to a high mass-productivity and an easy production of amine compounds represented by formulae (II), (III) and (IV).

In particular, the present invention concerns:
A process for producing an amine compound represented by formula (II) or formula (III), 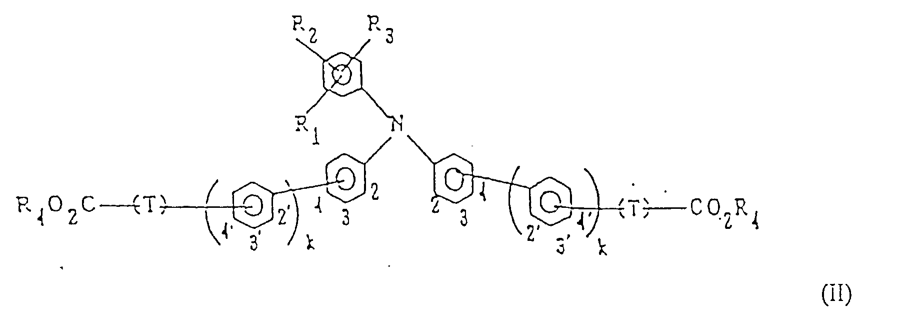 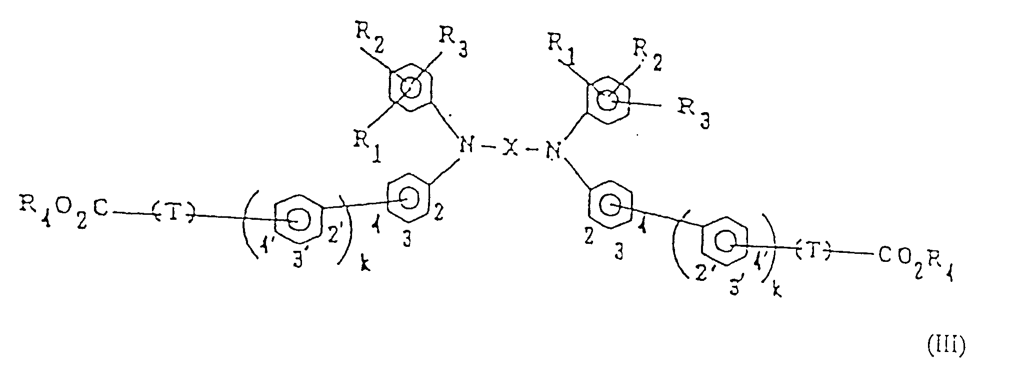 with the exception of N,N'-diphenyl-N,N'-bis [3- (2-ethoxycarbonylethyl)phenyl]-[1,1'-biphenyl] -4,4'-diamine; N,N'-diphenyl-N,N'-bis[4-(4-ethoxycarbonylethylphenyl)phenyl]-[1,1'-biphenyl]-4,4'-diamine, and N,N'-diphenyl-N,N'-bis[4-(4-ethoxycarbonylmethylphenyl)phenyl]-[1,1'-biphenyl]-4,4'-diamine, comprising reacting a compound represented by formula (I) 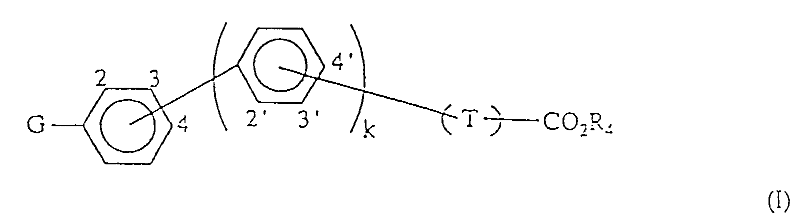 with a compound represented by formula (V) or formula (IX) 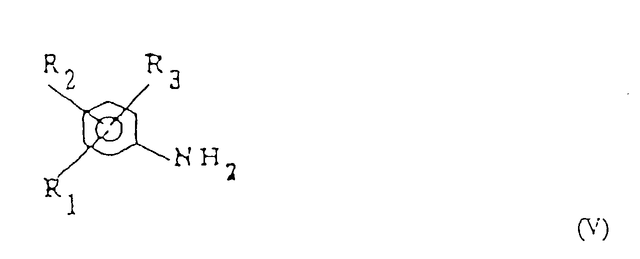 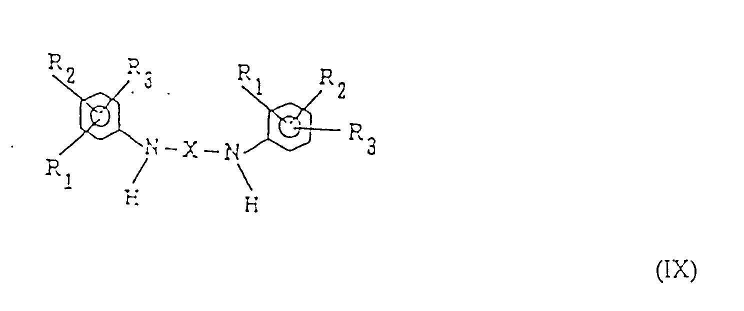 wherein R₁, R₂ or R₃ each independently represents a hydrogen atom, an alkyl group, an alkoxy group, a substituted amino group, a halogen atom or a substituted or unsubstituted aryl group; R₄ represents a hydrogen atom, an alkyl group, a substituted or unsubstituted aryl group or a substituted or unsubstituted aralkyl group; G represents a bromine atom or an iodine atom; X represents a substituted or unsubstituted divalent aryl group; k represents an integer of 0 or 1; and T represents a C₁₋₁₀ divalent hydrocarbon group that may be branched, in the presence of a copper catalyst.

Further, the invention relates to :

The process as defined above, wherein the compound represented by formula (I) is coupled with the compound represented by formula (V) to form the compound represented by formula (II).

The process as defined above, wherein the compound represented by formula (I) is coupled with the compound represented by formula (IX) to form the compound represented by formula (III).

The process as defined above, wherein said copper catalyst is selected from the group consisting of copper powder, cuprous chloride and copper sulfate.

The process as defined above, wherein 0.001 to 3 parts by weight copper catalyst based on 1 part by weight of the compound of formula (I) is used.

The process as defined above, wherein said reaction is conducted in the presence of a base.

The process as defined above, wherein 0.5 to 3 equivalents of the base to the compound of formula (I) is used.

The process as defined above, wherein said reaction is conducted at a temperature of from 100°C to 300°C.

In addition, the invention relates to :

A process for producing an amine compound represented by formula (III) or formula (IV), 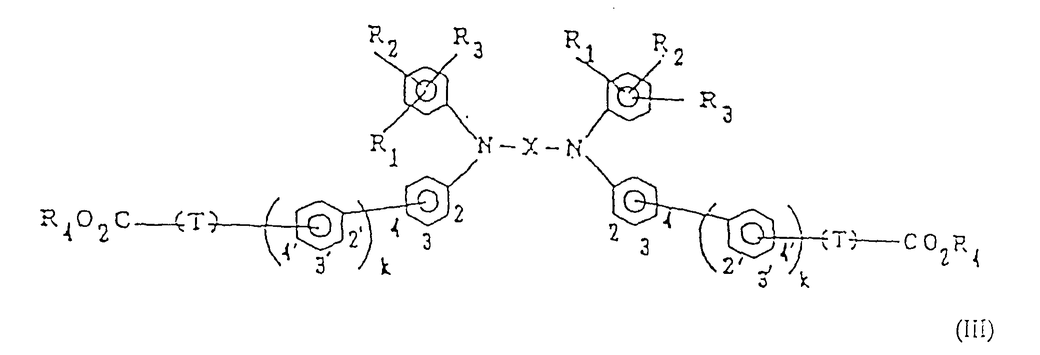 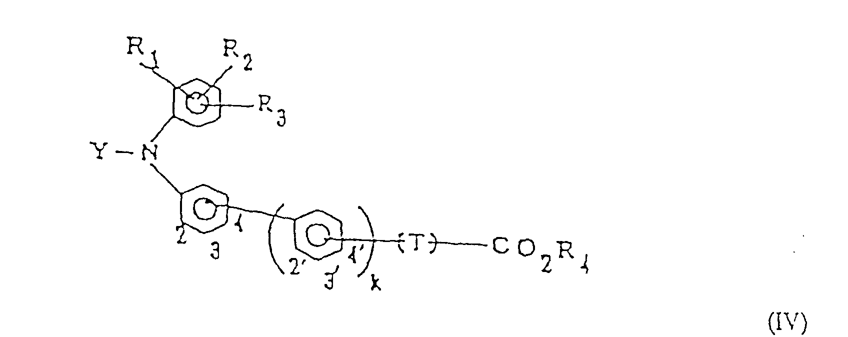 comprising:
1) reacting a compound represented by formula (I) 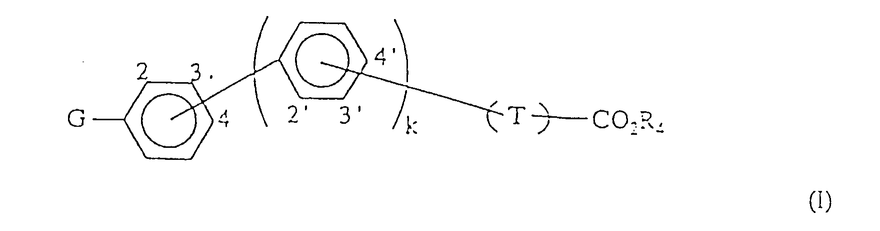 with a compound represented by formula (VI) 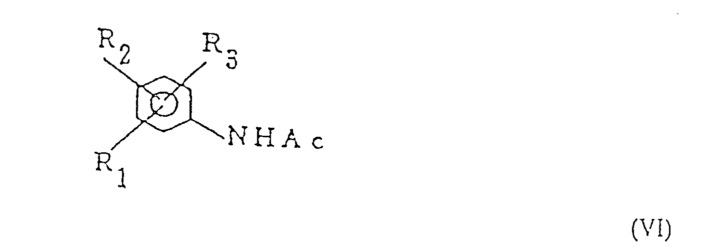 in the presence of a copper catalyst to obtain a reaction product;
2) hydrolyzing the reaction product to obtain a compound represented by formula (VII) or an ester thereof; 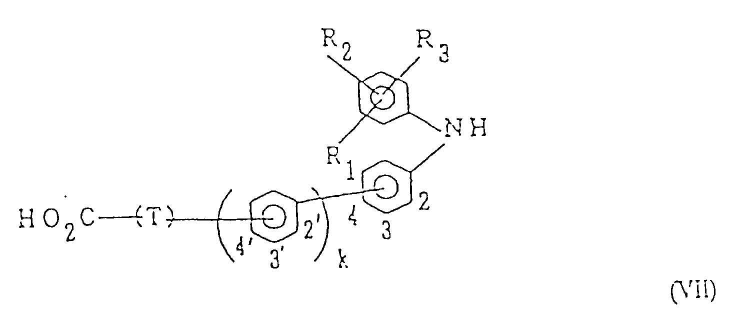 and
3) reacting the compound of formula (VII) or the ester thereof with either a compound of formula (VIII)

   G-X-G (VIII)

   or

   Y-G
wherein R₁, R₂ or R₃ each independently represents a hydrogen atom, an alkyl group, an alkoxy group, a substituted amino group, a halogen atom or a substituted or unsubstituted aryl group; R₄ represents a hydrogen atom, an alkyl group, a substituted or unsubstituted aryl group or a substituted or unsubstituted aralkyl group; G represents a bromine atom or an iodine atom; X represents a substituted or unsubstituted divalent aryl group; k represents an integer of 0 or 1; T represents a C₁₋₁₀ divalent hydrocarbon group that may be branched; and Y represents a substituted or unsubstituted aryl group in the presence of a copper catalyst.

Further, the invention relates to :

The process as immediately defined above, wherein the compound represented by formula (VII) or the ester thereof is coupled with the compound represented by formula (VIII) to form the compound represented by formula (III).

The process as immediately defined above, wherein the compound represented by formula (VII) or the ester thereof is coupled with Y-G to form the compound represented by formula (IV).

The process as immediately defined above, wherein the copper catalyst in one or both of steps 1) and 3) is selected from the group consisting of copper powder, cuprous chloride and copper sulfate.

The process as immediately defined above, wherein 0.001 to 3 parts by weight copper catalyst based on 1 part by weight of the compound of formula (I) is used.

The process as immediately defined above, wherein at least one step in the reaction is conducted in the presence of a base.

The process as immediately defined above, wherein in step 1) 0.5 to 3 equivalents of a base to the compound of formula (I) is used.

The process as immediately defined above, wherein the reaction of step 1) is conducted at a temperature of from 100°C to 300°C.

The process as immediately defined above, wherein said hydrolyzation reaction is conducted in the presence of a base in a solvent.

The process as immediately defined above, wherein during said hydrolyzation reaction 0.5 to 10 parts by weight solvent based on 1 part by weight of the compound of formula (I) is used.

The process as immediately defined above, wherein during said hydrolyzation reaction 0.2 to 5 parts by weight base based on 1 part by weight of the compound of formula (I) is used.

The process as immediately defined above, wherein said hydrolyzation reaction is conducted at a temperature of from 50°C to the boiling point of the solvent in an atmosphere of inert gas.

### BRIEF DESCRIPTION OF THE DRAWINGS

Fig. 1 illustrates IR spectrum of the amine compound (II-8) of Example 1.

Fig. 2 illustrates IR spectrum of the amine compound (II-23) of Example 2.

Fig. 3 illustrates IR spectrum of the amine compound (III-6) of Example 3.

Fig. 4 illustrates IR spectrum of the amine compound (III-7) of Reference Example 4.

Fig. 5 illustrates IR spectrum of the amine compound (III-9) of Example 5.

Fig. 6 illustrates IR spectrum of the amine compound (III-14) of Example 6.

Fig. 7 illustrates IR spectrum of the amine compound (III-16) of Example 7.

Fig. 8 illustrates IR spectrum of the amine compound (III-18) of Example 8.

Fig. 9 illustrates IR spectrum of the amine compound (III-19) of Example 9.

Fig. 10 illustrates IR spectrum of the amine compound (III-21) of Example 10.

Fig. 11 illustrates IR spectrum of the amine compound (III-24) of Example 11.

Fig. 12 illustrates IR spectrum of the amine compound (III-42) of Example 12.

Fig. 13 illustrates IR spectrum of the amine compound (III-53) of Reference Example 13.

Fig. 14 illustrates IR spectrum of the amine compound (III-55) of Reference Example 14.

Fig. 15 illustrates IR spectrum of the amine compound (IV-12) of Example 15.

### DETAILED DESCRIPTION OF THE INVENTION

Embodiments of the present invention will be further described hereinafter.

The synthesis of the compound represented by formula (I) to be used as a starting material of the amine compound represented by formula (II), (III) or (IV) will be described hereinafter.

Examples of the process for the synthesis of the compound represented by formula (I) include the following processes (1) and (2):
(1) A process which comprises the halogenation of a compound represented by formula (X): 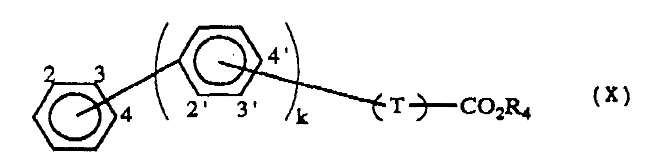 wherein R₄ represents a hydrogen atom, an alkyl group, a substituted or unsubstituted aryl group or a substituted or unsubstituted aralkyl group; and k represents an integer of 0 or 1.

In the foregoing general formula (X), T represents a C₁₋₁₀ divalent hydrocarbon group which may be branched. Specific examples of the structural formula of such a hydrocarbon group will be given below.

The arylamine skeleton in formula (II), (III) or (IV) may be bonded to either side of the T structure. For example, T-5(r) indicates that the arylamine skeleton is bonded to the right side of the structure T-5, and T-5(l) indicates that the arylamine skeleton is bonded to the left side of the structure T-5.

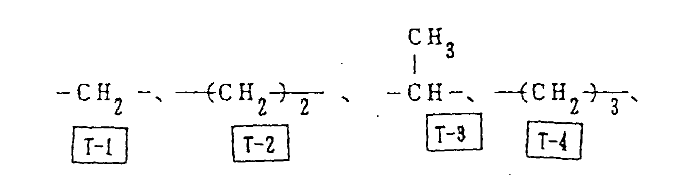

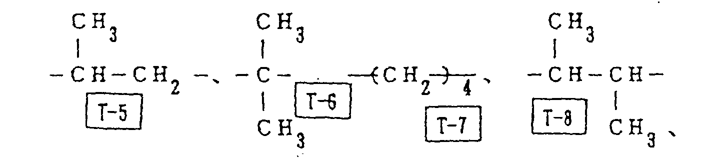

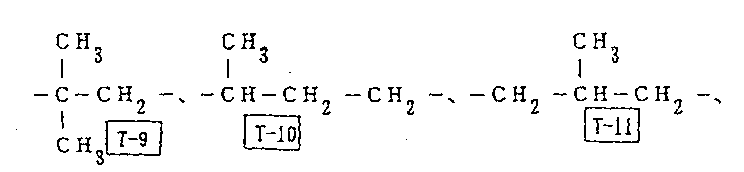

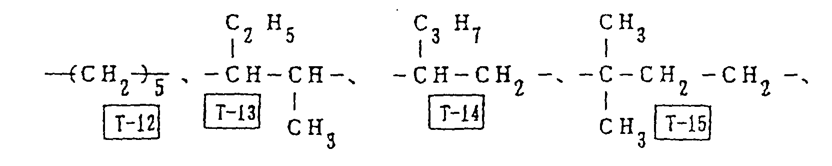

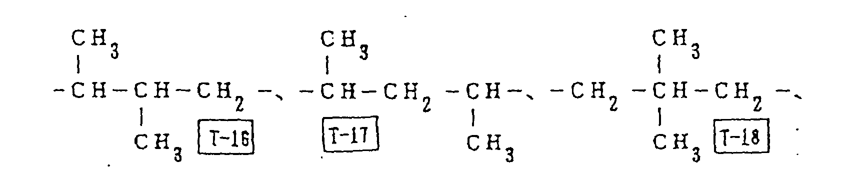

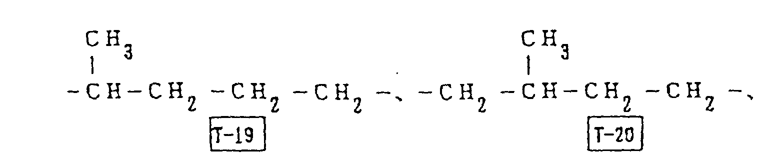

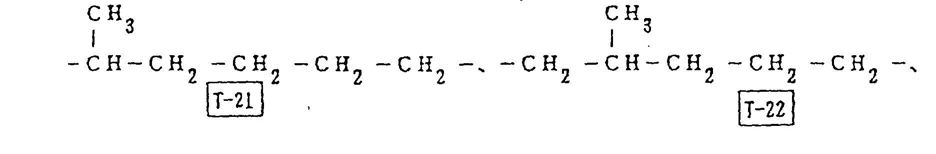

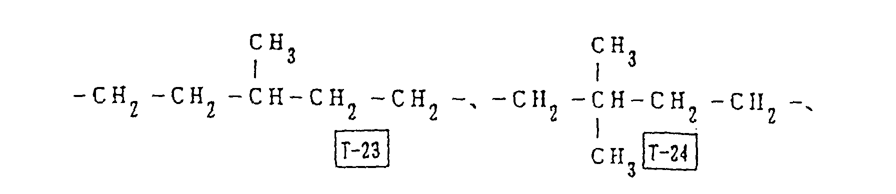

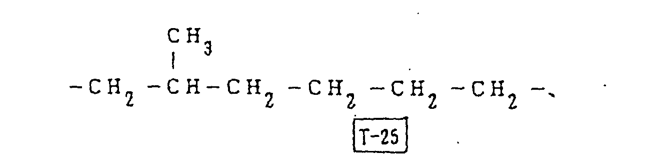

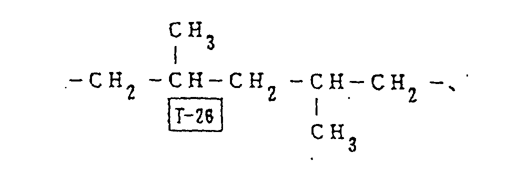

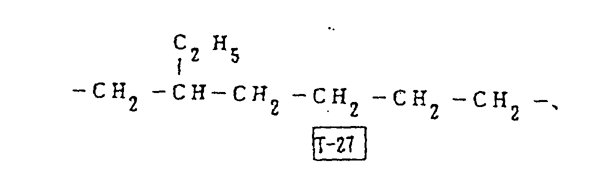

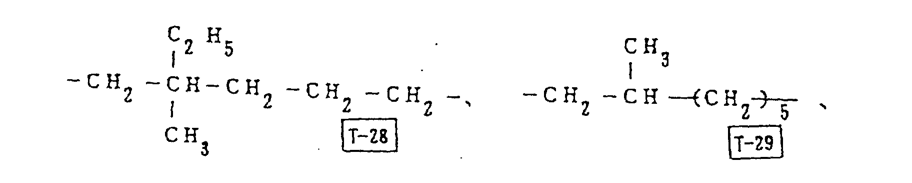

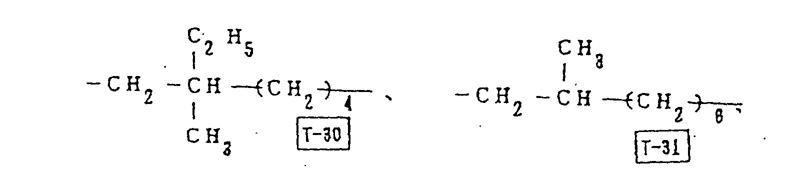

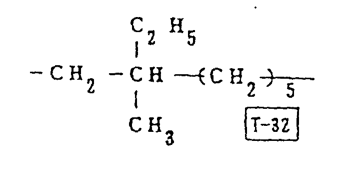

Specific examples of the compound represented by formula (I) will be given in Tables 1 and 2.

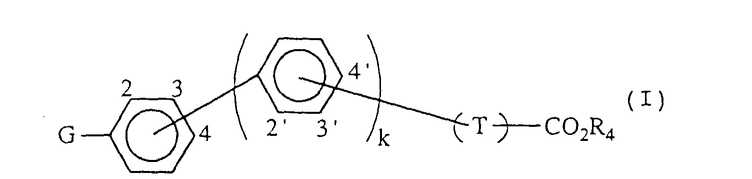

**TABLE 1**

| Compound No. | G | Bonding position | k | T | R₄ | Comppound No. | G | Bonding position | k | T | R₄ |
|---|---|---|---|---|---|---|---|---|---|---|---|
| I-1 | I | 2 | 0 | T-1 | H | I-16 | I | 4 | 0 | T-4 | H |
| I-2 | I | 3 | 0 | T-1 | H | I-17 | I | 4 | 0 | T-4 | H |
| I-3 | I | 4 | 0 | T-1 | H | I-18 | I | 4 | 0 | T-4 | H |
| I-4 | I | 2 | 0 | T-1 | C₂H₅ | I-19 | I | 4 | 0 | T-5(l) | H |
| I-5 | I | 3 | 0 | T-1 | C₂H₅ | I-20 | I | 4 | 0 | T-8 | H |
| I-6 | I | 4 | 0 | T-1 | C₂H₅ | I-21 | I | 4 | 0 | T-13(l) | H |
| I-7 | I | 2 | 0 | T-2 | H | I-22 | I | 4 | 0 | T-18 | H |
| I-8 | I | 3 | 0 | T-2 | H | I-23 | I | 4 | 0 | T-20(l) | H |
| I-9 | I | 4 | 0 | T-2 | H | I-24 | I | 4 | 0 | T-24(l) | H |
| I-10 | I | 2 | 0 | T-2 | C₂H₅ | I-25 | I | 4 | 0 | T-25(l) | H |
| I-11 | I | 3 | 0 | T-2 | C₂H₅ | I-26 | I | 3,3' | 1 | T-1 | H |
| I-12 | I | 4 | 0 | T-2 | C₂H₅ | I-27 | I | 4,4' | 1 | T-1 | H |
| I-13 | Br | 2 | 0 | T-2 | C₂H₅ | I-28 | I | 3,3' | 1 | T-1 | CH₃ |
| I-14 | Br | 3 | 0 | T-2 | C₂H₅ | I-29 | I | 4,4' | 1 | T-1 | CH₃ |
| I-15 | Br | 4 | 0 | T-2 | CH₃ | I-30 | I | 4,4' | 1 | T-1 | C₂H₅ |

**TABLE 2**

| Compound No. | G | Bonding position | k | T | R₄ |
|---|---|---|---|---|---|
| I-31 | I | 3,3' | 1 | T-2 | H |
| I-32 | I | 4,4' | 1 | T-2 | H |
| I-33 | I | 4,4' | 1 | T-2 | CH₃ |
| I-34 | I | 4,4' | 1 | T-2 | C₂H₅ |
| I-35 | Br | 4,4' | 1 | T-2 | H |
| I-36 | Br | 4,4' | 1 | T-2 | CH₃ |
| I-37 | Br | 4,4' | 1 | T-2 | C₂H₅ |
| I-38 | I | 4,4' | 1 | T-4 | H |
| I-39 | I | 4,4' | 1 | T-4 | CH₃ |
| I-40 | I | 4,4' | 1 | T-4 | C₂H₅ |
| I-41 | I | 4,4' | 1 | T-5(l) | H |
| I-42 | I | 3,3' | 1 | T-8 | C₆H₅ |
| I-43 | I | 3,3' | 1 | T-13(l) | H |
| I-44 | I | 4,4' | 1 | T-18 | CH₃ |
| I-45 | I | 4,4' | 1 | T-20(l) | H |
| I-46 | I | 4,4' | 1 | T-24(l) | C₂H₅ |
| I-47 | I | 4,4' | 1 | T-25(l) | H |

Bromides of these aromatic hydrocarbons can be obtained by bromiding these aromatic hydrocarbons directly with bromine in the presence of a catalyst such as bromine as described in Org. Syn., Coll. Vol. 1, 123, 1944. Further, iodides of these aromatic hydrocarbons can be obtained by any known method, e.g., by iodizing these aromatic hydrocarbons with iodic acid and iodine in the presence of a sulfuric acid catalyst in the mixture of water and acetic acid as described in Ann. 634, 84, 1960 or by iodizing these aromatic hydrocarbons with periodic acid and iodine in the presence of sulfuric acid catalyst in the mixture of water and acetic acid as described in "Journal of Japan Society of Chemistry", 92, 1021, 1971.

The iodization of aromatic hydrocarbons will be further described hereinafter with reference to the case where periodic acid and iodine are used as iodine sources.

An aromatic hydrocarbon and a predetermined amount of periodic acid, iodine and a catalyst are added to a mixture of water and acetic acid. The mixture is heated with stirring. The mixture of water and acetic acid to be used falls within a range of from 1 to 100 mℓ based on 1 part of the aromatic hydrocarbon. If the amount of the mixture of water and acetic acid falls below the above defined range, the reaction can hardly proceed smoothly. On the contrary, if the amount of the mixture of water and acetic acid exceeds the above defined range, the after treatment takes much time. Thus, the amount of the mixture of water and acetic acid is preferably from 2 to 30 mℓ. The ratio of water to acetic acid is 1 mℓ : 0.5 to 100 mℓ. If the ratio of water is excessive, the aromatic hydrocarbon cannot be dissolved in the solvent, making it difficult for the reaction to proceed. On the contrary, if the ratio of water is too small, side reactions can easily take place. Thus, the ratio of acetic acid is preferably from 0.5 to 10 mℓ, particularly from 0.8 to 8 mℓ based on 1 mℓ of water. Periodic acid oxidizes hydrogen iodide produced by the reaction to cause the reaction to proceed smoothly. The molar ratio of periodic acid to iodine is preferably not less than 1/3.5. Periodic acid may be used in the form of dihydrate or aqueous solution thereof. In order to accomplish the safety in the reaction, periodic acid is preferably used in the form of aqueous solution. As the catalyst there may be used sulfuric acid, p-toluenesulfonic acid, nitrosylsulfuric acid, mixture of sulfuric acid and nitric acid or peroxide such as peracetic acid and sodium persulfate. Sulfuric acid is desirable in that it is inexpensive and doesn't cause any reaction such as nitration. The amount of the catalyst to be used is not less than 0.01 parts, preferably not less than 0.1 parts based on 1 part of aromatic hydrocarbon.

The foregoing iodization reaction is effected under heating with stirring. Iodine is sublimable and deposited at the upper part of the reactor. Therefore, it is desirable that the reaction system is heated to a temperature of not less than 80°C so that the solvent is refluxed to inhibit the deposition of iodine. The termination of the reaction can be confirmed by the disappearance of the color of iodine. After the completion of the reaction, the reaction system is allowed to cool to room temperature. The resulting product is separated and then dissolved in a proper organic solvent such as methylene chloride, toluene and ethyl acetate. The resulting organic phase is washed with a dilute aqueous solution of sodium thiosulfate or sodium carbonate, thoroughly washed with water, and then dried to distill the solvent away. The reaction residue is then recrystallized from a proper organic solvent such as ethyl acetate, toluene, mixture of ethyl acetate and ethanol and mixture of toluene and ethanol to obtain an iodized aromatic hydrocarbon. As the organic solvent there may be used any of these organic solvents in an arbitrary amount. The iodized aromatic hydrocarbon thus obtained can be used in the subsequent coupling reaction as it is or after esterified.
(2) A process which comprises the reaction of a compound represented by formula (XI) with a malonic acid ester, and then decarboxylizing the reaction product under heating to synthesize the compound represented by formula (I): 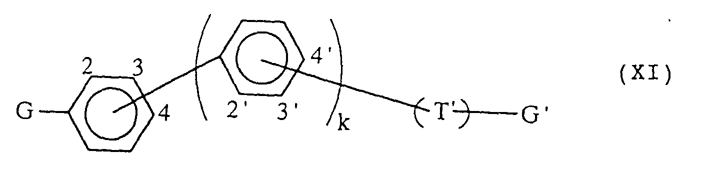 wherein G and G' each represents a bromine atom or an iodine atom; k represents an integer of 0 or 1; and T' represents a C₁₋₉ divalent hydrocarbon group which may be branched, which undergoes carbon-addition reaction with a malonic acid ester to have the structure of T.

The foregoing reaction can be effected in accordance with the method described in, e.g., "Jikken Kagaku Koza (Institute of Experimental Chemistry)", Vol. 4, 22, 59, (1992). The foregoing reaction will be further described hereinafter.

Malonic acid diethyl or malonic acid dimethyl is dissolved in ethanol or methanol. To the solution is then gradually added metallic sodium or sodium alcoholate in about the same equivalent. The mixture is then thoroughly stirred. To the mixture is then added slowly dropwise an ethanol or methanol solution of the compound represented by formula (XI). Ethanol or methanol may be used in an amount of from 1 to 20 parts based on 1 part of malonic acid diethyl or malonic acid dimethyl. Alternatively, malonic acid diethyl may be excessively used instead of ethanol or methanol. During the dropwise addition of the compound represented by formula (XI), the chemical reaction is preferably effected under cooling in an ice bath or the like so that it doesn't proceed with great violence. After the completion of the dropwise addition, the reaction system may be heated to complete the reaction. Further, the reaction is preferably effected in an atmosphere of inert gas such as nitrogen.

After the completion of the foregoing reaction, the reaction product is thoroughly washed with water, and then purified by distillation or the like to obtain a diester represented by formula (XII). The diester thus obtained is then heat-decarboxilized with water, LiCl and dimethyl sulfoxide (DMSO) to obtain the compound represented by formula (X). In this case, water is used in almost the same amount as the diester represented by formula (XII). LiCl is used in about twice the amount of the diester represented by formula (XII). DMSO is used in an amount of from 1 to 50 parts, preferably from 2 to 30 parts based on 1 part of diester. The decarboxylation reaction is preferably effected at a temperature between 100°C and the boiling point of DMSO in an atmosphere of inert gas such as nitrogen. After the completion of the reaction, the reaction product is thoroughly washed with water, and then purified by distillation or the like to obtain the compound represented by formula (X). 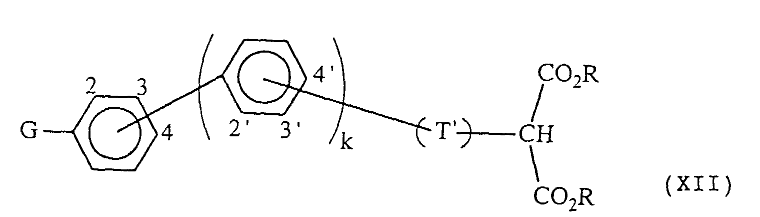 wherein G represents a bromine atom or an iodine atom; k represents an integer of 0 or 1; T' represents a C₁₋₉ divalent hydrocarbon group which may be branched, which undergoes carbon-addition reaction with a malonic acid ester to have the structure of T; and R represents a methyl group or an ethyl group.

The process for the preparation of the amine compound represented by formula (II) of the present invention will be described hereinafter.

In the present invention, the amine compound represented by formula (II) can be obtained by the coupling reaction of the compound represented by formula (I) with the compound represented by formula (V) in the presence of a copper catalyst:

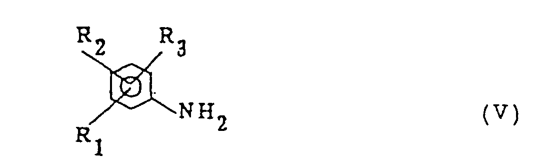

Examples of the compound represented by formula (V) to be used in this coupling reaction include aniline, o-toluidine, m-toluidine, p-toluidine, 2,3-xylidine, 2,4-xylidine, 2,5-xylidine, 2,6-xylidine, 3,4-xylidine, 3,5-xylidine, 2,4,6-trimethylaniline, o-biphenylamine, m-biphenylamine, and p-biphenylamine.

In the coupling reaction of the compound represented by the foregoing amine compound, a copper catalyst, a base, and optionally a solvent are used. The compound represented by formula (I) is used in 2 to 5 equivalents, preferably 2.2 to 4 equivalents to the amine compound. As the copper catalyst there may be used copper powder, cuprous chloride, copper sulfate or the like. The amount of the copper catalyst to be used is from 0.001 to 3 parts by weight, preferably from 0.01 to 2 parts by weight based on 1 part by weight of the compound represented by formula (I). As the base there may be used sodium hydroxide, potassium hydroxide, sodium carbonate, potassium carbonate or the like. The amount of the base to be used is from 0.5 to 3 equivalents, preferably from 0.7 to 2 equivalents to the compound represented by formula (I). As the solvent there is preferably used a high boiling water-insoluble hydrocarbon solvent such as n-tridecane and tetralin. The solvent is used in an amount of from 0.1 to 3 parts by weight, preferably from 0.2 to 2 parts by weight based on 1 part by weight of the compound represented by formula (I).

This reaction is effected at a temperature of from 100°C to 300°C, preferably from 150°C to 270°C, more preferably from 180°C to 250°C in an atmosphere of inert gas such as nitrogen with a sufficiently efficient stirring. Further, this reaction is preferably effected while water produced is being removed. After the completion of the reaction, the reaction product is dissolved in a solvent such as toluene, Isoper and n-tridecane. If necessary, the solution is rinsed or filtered to remove undesirable substances therefrom. The solution is then subjected to column purification with silica gel, alumina, active clay, activated charcoal or the like. Alternatively, these adsorbents are added to the solvent to adsorb undesirable substances. The product is then recrystallized from a proper solvent such as ethanol, ethyl acetate and toluene to undergo purification.

The process for the preparation of the amine compound represented by formula (III) of the present invention will be described hereinafter.

In the present invention, the compound represented by formula (III) can be obtained by allowing the compound represented by formula (I) and the compound represented by formula (VI) to undergo coupling reaction in the presence of a copper catalyst, hydrolyzing the reaction product to obtain a compound represented by formula (VII), and then allowing the compound represented by formula (VII) or the ester thereof to undergo coupling reaction with a compound represented by formula (VIII) in the presence of a copper catalyst.

Examples of the compound represented by formula (VI) include acetylation product of aniline, o-toluidine, m-toluidine, p-toluidine, 2,3-xylidine, 2,4-xylidine, 2,5-xylidine, 2,6-xylidine, 3,4-xylidine, 3,5-xylidine, 2,4,6-trimethylaniline, o-biphenylamine, m-biphenylamine and p-biphenylamine.

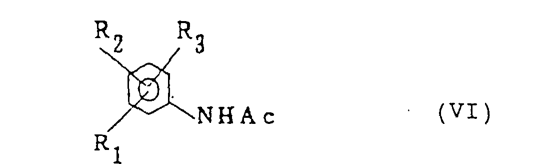

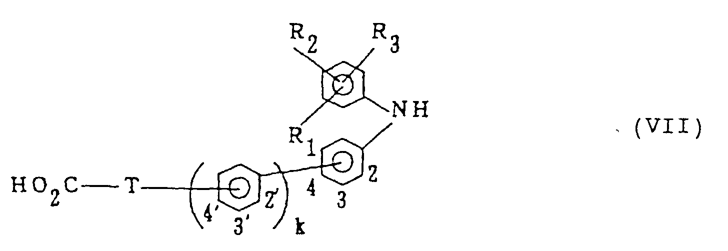

G-X-G (VIII)

wherein R₁ to R₃ each independently represent a hydrogen atom, an alkyl group, an alkoxy group, a substituted amino group, a halogen atom or a substituted or unsubstituted aryl group; G represents a bromine atom or an iodine atom; X represents a substituted or unsubstituted divalent aryl group; k represents an integer of 0 or 1; and T represents a C₁₋₁₀ divalent hydrocarbon group which may be branched.

Examples of the group represented by X include those selected from the following groups (1) to (7): 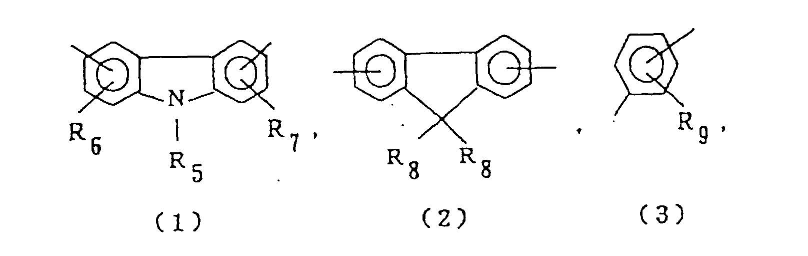 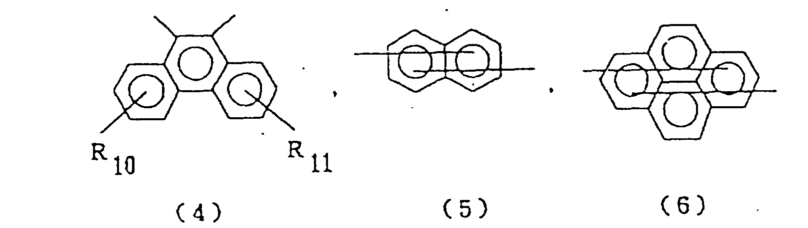 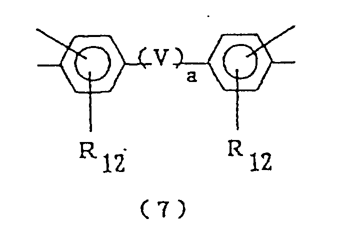 wherein R₅ represents a hydrogen atom, a C₁₋₄ alkyl group, a substituted or unsubstituted phenyl group or a substituted or unsubstituted aralkyl group; R₆ to R₁₂ each represents a hydrogen atom, a C₁₋₄ alkyl group, a C₁₋₄ alkoxy group, a substituted or unsubstituted phenyl group, a substituted or unsubstituted aralkyl group or a halogen atom; and a represents an integer of 0 or 1.

V represents a group selected from the following groups (8) to (17). 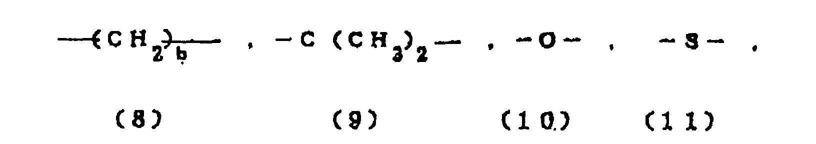 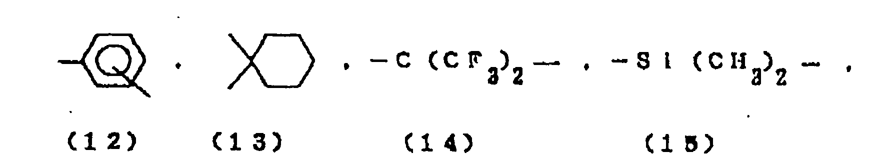 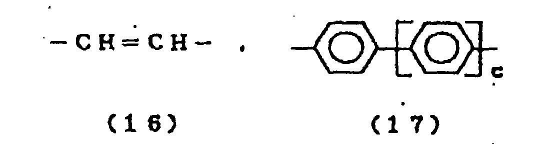 wherein b represents an integer of from 1 to 10; and c represents an integer of from 1 to 3.

In the foregoing coupling reaction, the compound represented by formula (I) is used in an amount of from 0.5 to 1.5 equivalents, preferably from 0.7 to 1.2 equivalents to the acetylated product. As the copper catalyst there may be used copper powder, cuprous chloride, copper sulfate or the like. The amount of the copper catalyst to be used is from 0.001 to 3 parts by weight, preferably from 0.01 to 2 parts by weight based on 1 part by weight of the compound represented by formula (I). As the base there may be used sodium hydroxide, potassium hydroxide, sodium carbonate, potassium carbonate or the like. The amount of the base to be used is from 0.5 to 3 equivalents, preferably from 0.7 to 2 equivalents to the compound represented by formula (I).

A solvent is not necessarily needed in this reaction. Preferred examples of the solvent, if used, include a high boiling water-insoluble hydrocarbon solvent such as n-tridecane and tetralin. Such a solvent may be used in an amount of from 0.1 to 3 parts by weight, preferably from 0.2 to 2 parts by weight based on 1 part by weight of the compound represented by formula (I). This reaction is effected at a temperature of from 100°C to 300°C, preferably from 150°C to 270°C, more preferably from 180°C to 250°C in an atmosphere of inert gas such as nitrogen with a sufficiently efficient stirring. Further, this reaction is preferably effected while water produced is being removed. After the completion of the reaction, the reaction system is cooled as necessary. The reaction system is then hydrolyzed with a base such as sodium hydroxide and potassium hydroxide in a solvent such as methanol, ethanol, n-octanol, ethylene glycol, propylene glycol and glycerin. The amount of the solvent to be used is from 0.5 to 10 parts by weight, preferably from 1 to 5 parts by weight based on 1 part by weight of the compound represented by formula (I). The amount of the base to be used is from 0.2 to 5 parts by weight, preferably from 0.3 to 3 parts by weight based on 1 part by weight of the compound represented by formula (I).

For the hydrolyzation reaction, the solvent and the base are directly charged into the reaction vessel after the coupling reaction. The hydrolyzation reaction is effected at a temperature of from 50°C to the boiling point of the solvent in an atmosphere of inert gas such as nitrogen with a sufficiently efficient stirring. In the foregoing coupling reaction, a carboxylate is produced and solidified. Further, in the post-treatment, the reaction system is poured into water, and then neutralized with hydrochloric acid or the like to free a compound represented by formula (VII). Therefore, as the solvent to be used in this reaction, a water-soluble solvent having a boiling point of not lower than 150°C such as ethylene glycol, propylene glycol and glycerin is particularly preferred. After the completion of the hydrolyzation reaction, the reaction product is poured into water, and then neutralized with hydrochloric acid or the like to free a compound represented by formula (VII) which is then thoroughly washed. The compound thus obtained is then dissolved in a proper solvent. The solution is then subjected to column purification with silica gel, alumina, active clay, activated charcoal or the like. Alternatively, such an adsorbent is added to the solvent to adsorb undesirable substances. The product thus purified is then recrystallized from a proper solvent such as ethanol, ethyl acetate and toluene to undergo further purification. Alternatively, the product may be esterified to a methyl ester or ethyl ester which is then subjected to the similar purification.

The compound represented by formula (VII) or the ester thereof and the compound represented by formula (VIII) thus obtained may be then allowed to undergo coupling reaction in the presence of a copper catalyst to obtain a compound represented by formula (III). In this coupling reaction, the compound represented by formula (VII) or the ester thereof is used in 1.5 to 5 equivalents, preferably 1.7 to 4 equivalents to the compound represented by formula (VIII). As the copper catalyst there may be used copper powder, cuprous chloride, copper sulfate or the like. The amount of the copper catalyst to be used is from 0.001 to 3 parts by weight, preferably from 0.01 to 2 parts by weight based on 1 part by weight of the compound represented by formula (VIII). As the base there may be used sodium hydroxide, potassium hydroxide, sodium carbonate, potassium carbonate or the like. The amount of the base to be used is from 1 to 6 equivalents, preferably from 1.4 to 4 equivalents to the compound represented by formula (VIII). A solvent may be used as necessary. Preferred examples of the solvent, if used, include a high boiling water-insoluble hydrocarbon solvent such as n-tridecane and tetralin. The solvent is used in an amount of from 0.1 to 3 parts by weight, preferably from 0.2 to 2 parts by weight based on 1 part by weight of the compound represented by formula (VIII). This reaction is effected at a temperature of from 100°C to 300°C, preferably from 150°C to 270°C, more preferably from 180°C to 250°C in an atmosphere of inert gas such as nitrogen with a sufficiently efficient stirring. Further, this reaction is preferably effected while water produced is being removed. After the completion of the reaction, the reaction product is dissolved in a solvent such as toluene, Isoper and n-tridecane. If necessary, the solution is rinsed or filtered to remove undesirable substances therefrom. The solution is then subjected to column purification with silica gel, alumina, active clay, activated charcoal or the like. Alternatively, these adsorbents are added to the solvent to adsorb undesirable substances. The product is then recrystallized from a proper solvent such as ethanol, ethyl acetate and toluene to undergo purification.

Another process for the preparation of the amine compound represented by formula (III) of the present invention will be described hereinafter.

In the present invention, the amine compound represented by formula (III) can be obtained by, as another synthesis method, allowing the compound represented by formula (I) and a diamine compound represented by formula (IX) to undergo coupling reaction in the presence of a copper catalyst: 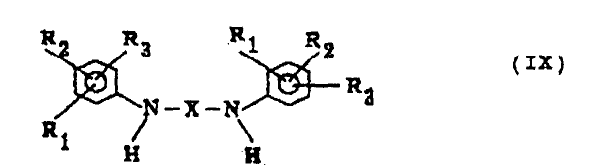 wherein R₁ to R₃, and X are as defined above.

In the foregoing coupling reaction, the compound represented by formula (I) and the diamine compound represented by formula (IX) are reacted in the presence of a copper catalyst and base, optionally in a solvent. The compound represented by formula (I) is used in 2 to 5 equivalents, preferably 2.2 to 4 equivalents to the diamine compound. As the copper catalyst there may be used copper powder, cuprous chloride, copper sulfate or the like. The amount of the copper catalyst to be used is from 0.001 to 3 parts by weight, preferably from 0.01 to 2 parts by weight based on 1 part by weight of the compound represented by formula (I). As the base there may be used sodium hydroxide, potassium hydroxide, sodium carbonate, potassium carbonate or the like. The amount of the base to be used is from 0.5 to 3 equivalents, preferably from 0.7 to 2 equivalents to the compound represented by formula (I). As the solvent there is preferably used a high boiling water-insoluble hydrocarbon solvent such as n-tridecane and tetralin. The solvent is used in an amount of from 0.1 to 3 parts by weight, preferably from 0.2 to 2 parts by weight based on 1 part by weight of the compound represented by formula (I). This reaction is effected at a temperature of from 100°C to 300°C, preferably from 150°C to 270°C, more preferably from 180°C to 250°C in an atmosphere of inert gas such as nitrogen with a sufficiently efficient stirring. Further, this reaction is preferably effected while water produced is being removed. After the completion of the reaction, the reaction product is dissolved in a solvent such as toluene, Isoper and n-tridecane. If necessary, the solution is rinsed or filtered to remove undesirable substances therefrom. The solution is then subjected to column purification with silica gel, alumina, active clay, activated charcoal or the like. Alternatively, these adsorbents are added to the solvent to adsorb undesirable substances. The product is then recrystallized from a proper solvent such as ethanol, ethyl acetate and toluene to undergo purification.

The process for the preparation of an amine compound represented by the general formula (IV) of the present invention will be described hereinafter.

In the present invention, the compound represented by the general formula (IV) can be obtained by the coupling reaction of a compound represented by the general formula (VII) or ester thereof with a compound represented by formula (X) in the presence of a copper catalyst:

Y - G (X)

wherein Y represents a substituted or unsubstituted aryl group; and G represents a bromine atom or iodine atom.

In the foregoing coupling reaction, the compound represented by formula (VII) or an ester thereof is reacted with the compound represented by formula (X) in an amount of about 1 equivalent in the presence of a copper catalyst and a base, optionally in a solvent. The compound represented by formula (X) is used in 0.5 to 1.5 equivalents, preferably 0.7 to 1.3 equivalents to the compound represented by formula (VII) or the ester thereof. As the copper catalyst there may be used copper powder, cuprous chloride, copper sulfate or the like. The amount of the copper catalyst to be used is from 0.001 to 3 parts by weight, preferably from 0.01 to 2 parts by weight based on 1 part by weight of the compound represented by formula (X). As the base there may be used sodium hydroxide, potassium hydroxide, sodium carbonate, potassium carbonate or the like. The amount of the base to be used is from 0.5 to 3 equivalents, preferably from 0.7 to 2 equivalents to the compound represented by the general formula (X). As the solvent there is preferably used a high boiling water-insoluble hydrocarbon solvent such as n-tridecane and tetralin. The solvent is used in an amount of from 0.1 to 3 parts by weight, preferably from 0.2 to 2 parts by weight based on 1 part by weight of the compound represented by formula (X).

This reaction is effected at a temperature of from 100°C to 300°C, preferably from 150°C to 270°C, more preferably from 180°C to 250°C in an atmosphere of inert gas such as nitrogen with a sufficiently efficient stirring. Further, this reaction is preferably effected while water produced is being removed. After the completion of the reaction, the reaction product is dissolved in a solvent such as toluene, Isoper and n-tridecane. If necessary, the solution is rinsed or filtered to remove undesirable substances therefrom. The solution is then subjected to column purification with silica gel, alumina, active clay, activated charcoal or the like. Alternatively, these adsorbents are added to the solvent to adsorb undesirable substances. The product is then recrystallized from a proper solvent such as ethanol, ethyl acetate and toluene to undergo purification to obtain a purified amine compound represented by formula (IV).

Specific examples of the amine compound represented by formula (II) obtained in the present invention will be given in Tables 3 and 4, but the present invention should not be construed as being limited thereto.

**TABLE 3**

| Compound No. | R₁ | R₂ | R₃ | R₄ | Bonding position | k | T |
|---|---|---|---|---|---|---|---|
| II-1 | H | H | H | CH₃ | 4 | 0 | T-1 |
| II-2 | H | H | H | C₂H₅ | 4 | 0 | T-1 |
| II-3 | H | 4-CH₃ | H | C₂H₅ | 4 | 0 | T-1 |
| II-4 | 3-CH₃ | 4-CH₃ | H | C₂H₅ | 4 | 0 | T-1 |
| II-5 | H | H | H | CH₃ | 3 | 0 | T-2 |
| II-6 | H | H | H | C₂H₅ | 3 | 0 | T-2 |
| II-7 | H | 4-CH₃ | H | C₂H₅ | 3 | 0 | T-2 |
| II-8 | 3-CH₃ | 4-CH₃ | H | C₂H₅ | 3 | 0 | T-2 |
| II-9 | 2-CH₃ | 4-CH₃ | 6-CH₃ | C₂H₅ | 3 | 0 | T-2 |
| II-10 | H | 4-C₆H₅ | H | CH₃ | 4 | 0 | T-2 |
| II-11 | 3-CH₃ | 4-CH₃ | H | CH₃ | 4 | 0 | T-5(l) |
| II-12 | H | H | H | CH₃ | 3 | 0 | T-5(l) |
| II-13 | 3-CH₃ | 4-CH₃ | H | CH₃ | 3 | 0 | T-8 |
| II-14 | 3-CH₃ | 4-CH₃ | H | CH₃ | 3 | 0 | T-25(l) |
| II-15 | H | H | H | CH₃ | 4 | 0 | T-5(r) |
| II-16 | H | H | H | CH₃ | 4,4' | 1 | T-1 |
| II-17 | H | H | H | C₂H₅ | 4,4' | 1 | T-1 |
| II-18 | H | 4-CH₃ | H | C₂H₅ | 4,4' | 1 | T-1 |
| II-19 | 3-CH₃ | 4-CH₃ | H | C₂H₅ | 4,4' | 1 | T-1 |
| II-20 | H | H | H | CH₃ | 4,4' | 1 | T-2 |

**TABLE 4**

| Compound No. | R₁ | R₂ | R₃ | R₄ | Bonding position | k | T |
|---|---|---|---|---|---|---|---|
| II-21 | H | H | H | C₂H₅ | 4,4' | 1 | T-2 |
| II-22 | H | 4-CH₃ | H | C₂H₅ | 4,4' | 1 | T-2 |
| II-23 | 3-CH₃ | 4-CH₃ | H | C₂H₅ | 4,4' | 1 | T-2 |
| II-24 | 2-CH3₃ | 4-CH₃ | 6-CH₃ | C₂H₅ | 4,4' | 1 | T-2 |
| II-25 | H | 4-C₆H₅ | H | CH₃ | 4,4' | 1 | T-2 |
| II-26 | 3-CH₃ | 4-CH₃ | H | CH₃ | 4,4' | 1 | T-5(l) |
| II-27 | H | H | H | CH₃ | 3,3' | 1 | T-5(l) |
| II-28 | 3-CH₃ | 4-CH₃ | H | CH₃ | 3,3' | 1 | T-8 |
| II-29 | 3-CH₃ | 4-CH₃ | H | CH₃ | 3,3' | 1 | T-25(l) |
| II-30 | H | H | H | CH₃ | 4,4' | 1 | T-5(r) |

Specific examples of the amine compound represented by formula (III) obtained in the present invention will be given in Tables 5 to 14 below, but the present invention should not be construed as being limited thereto.

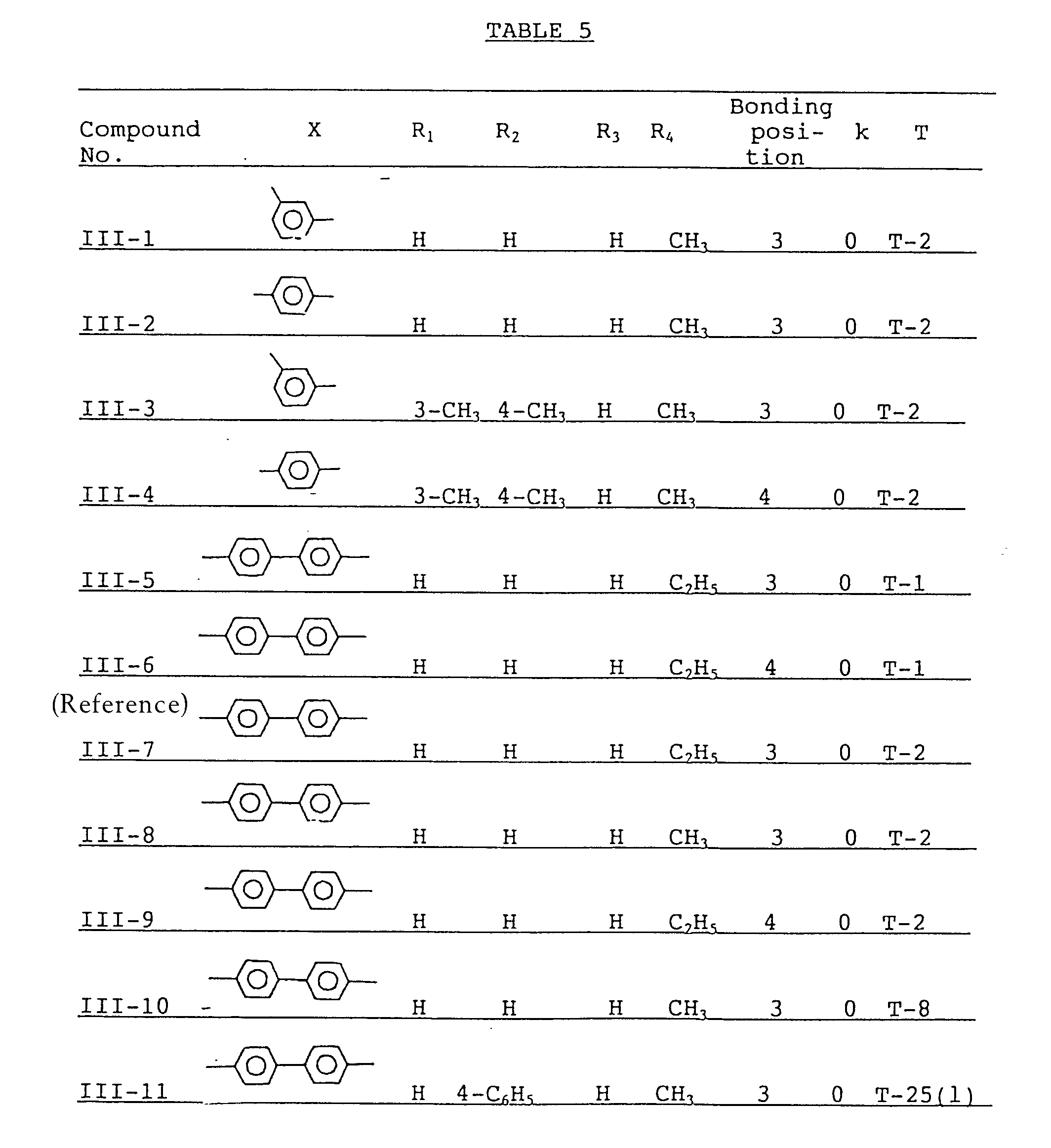

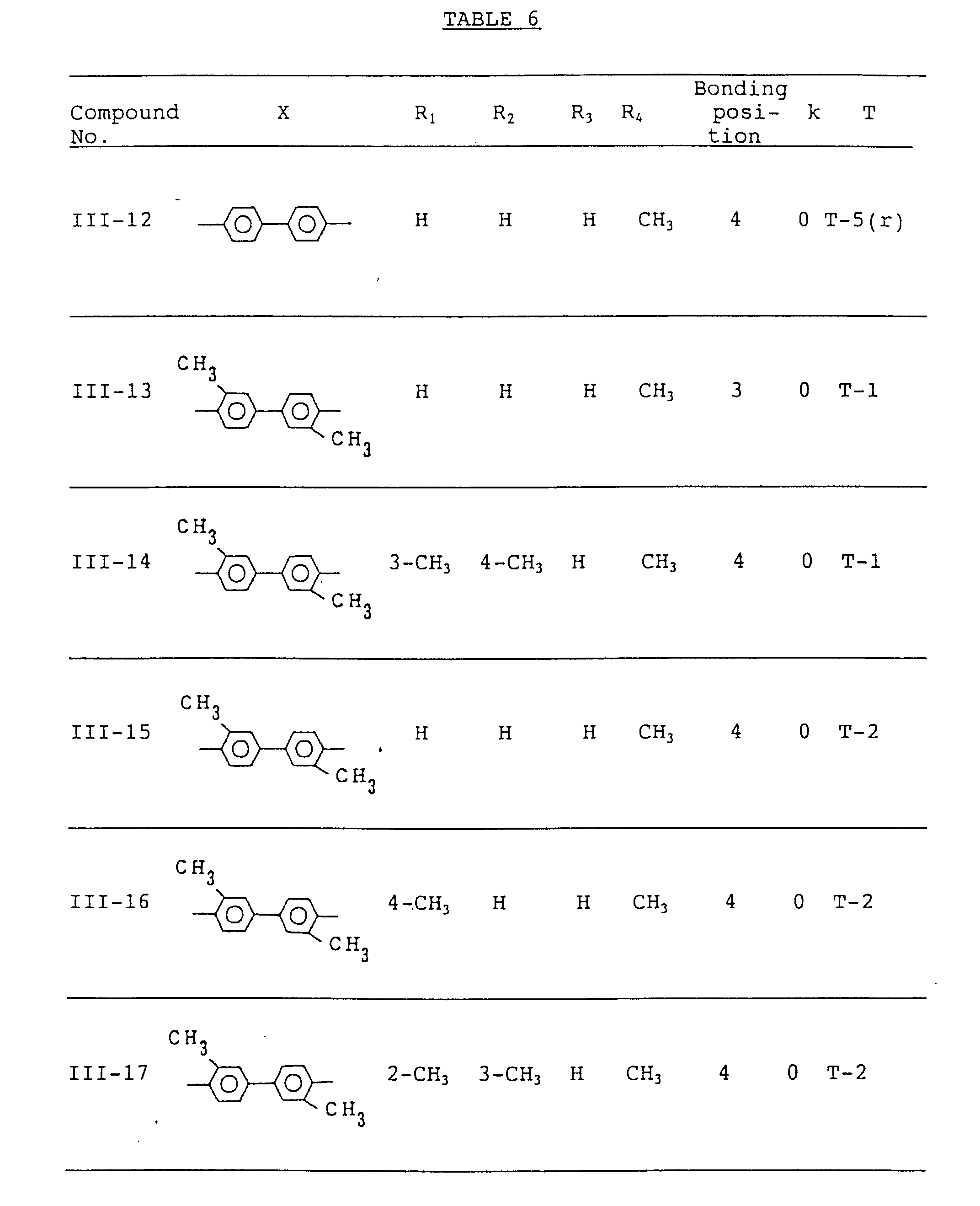

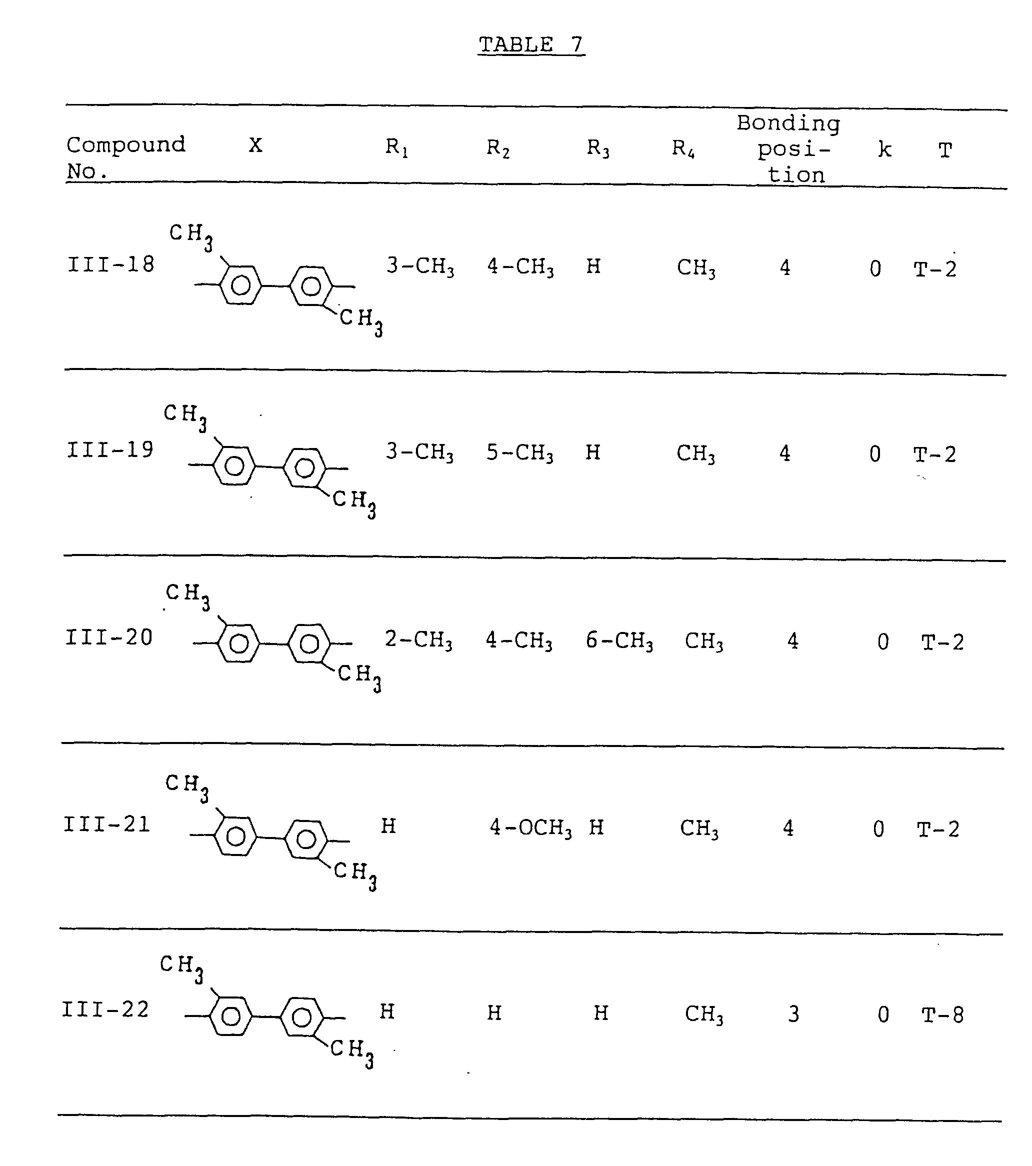

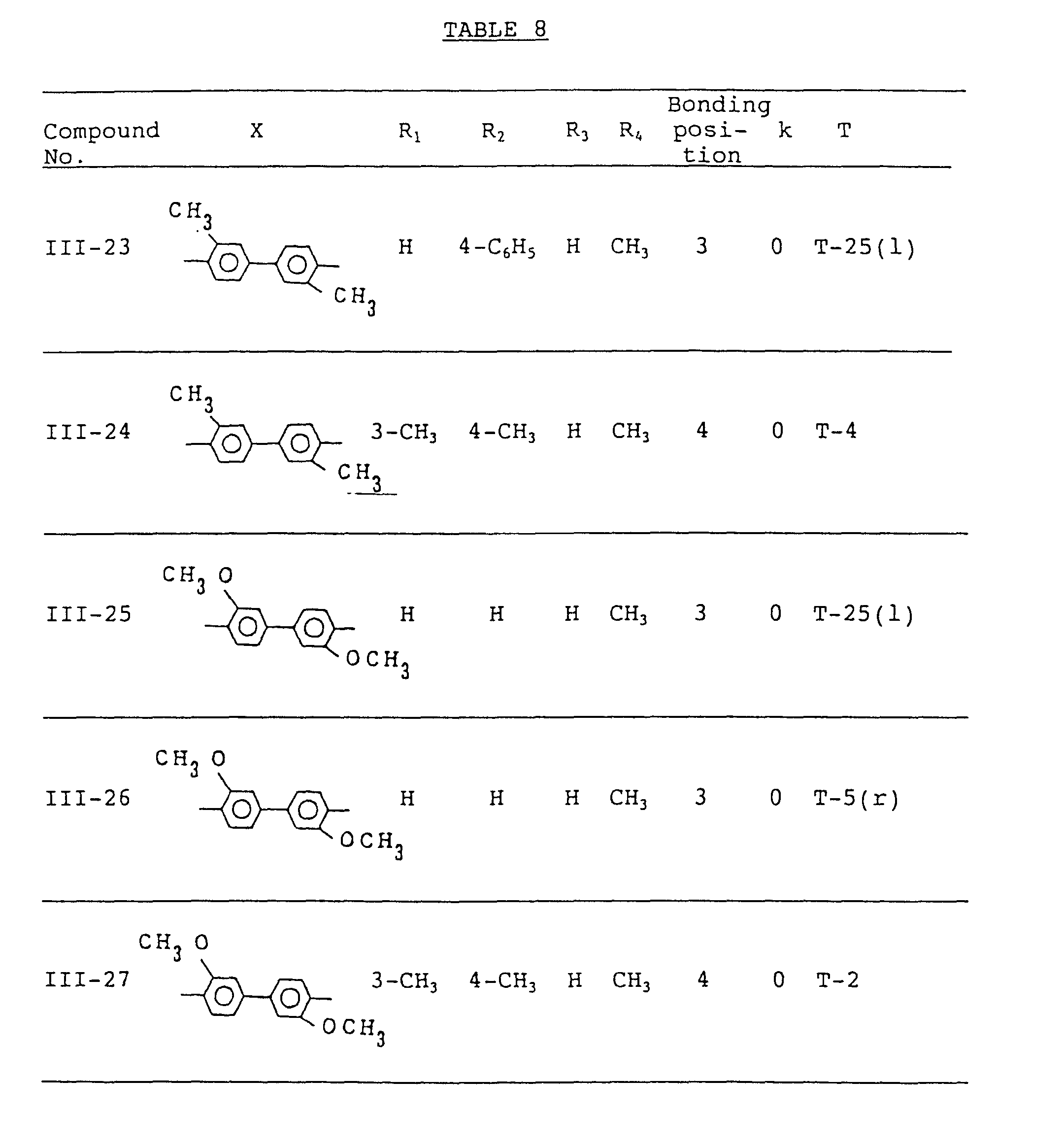

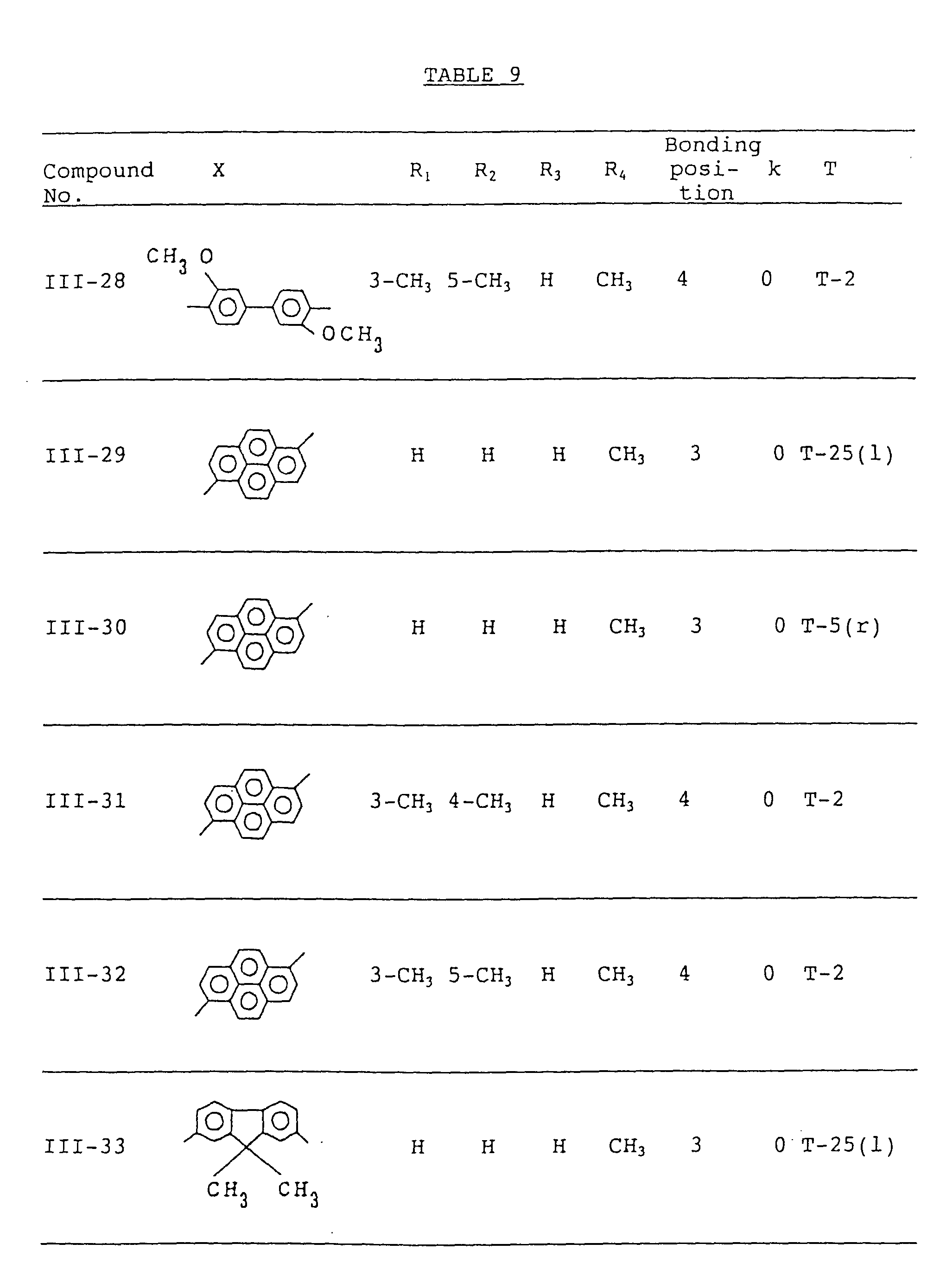

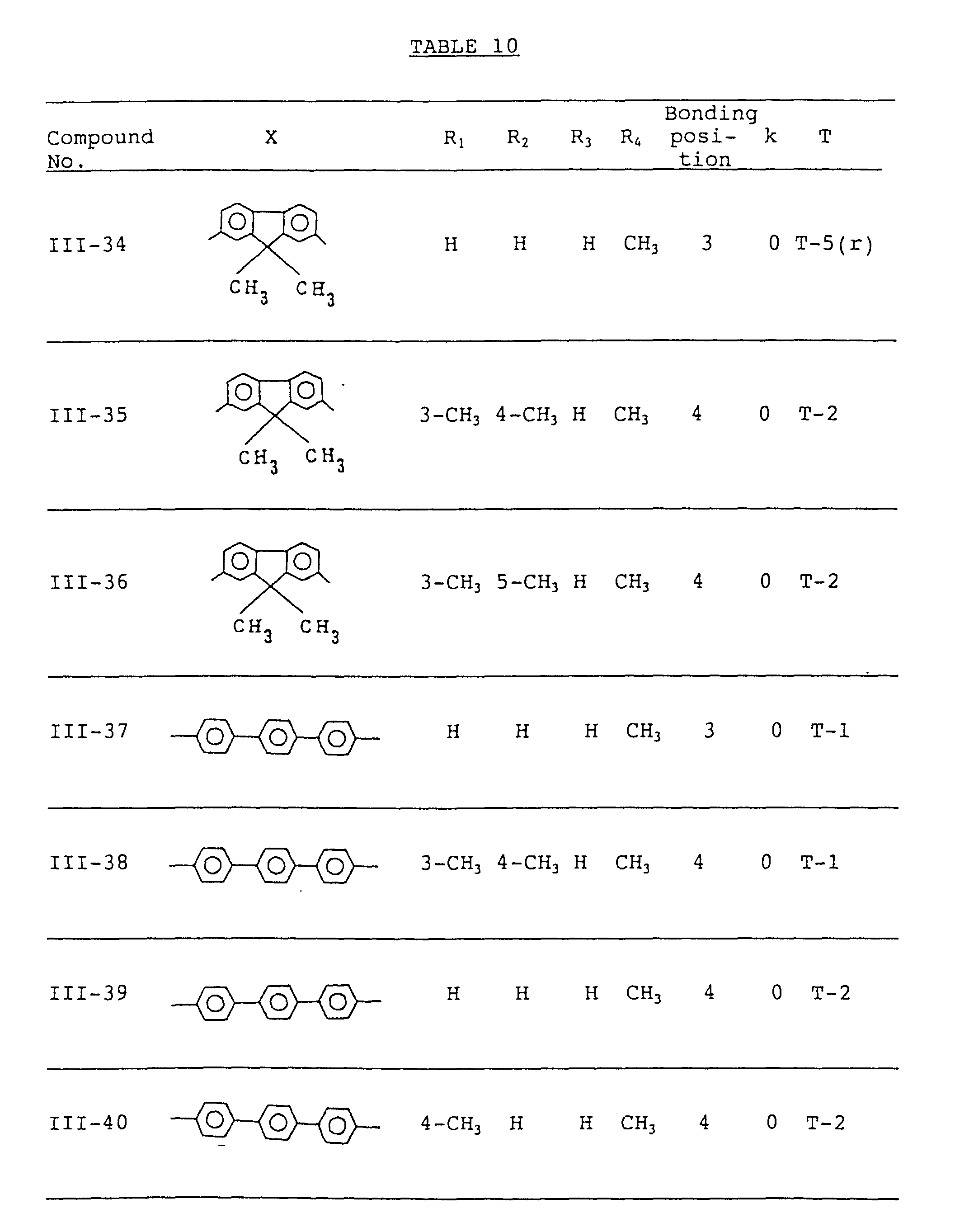

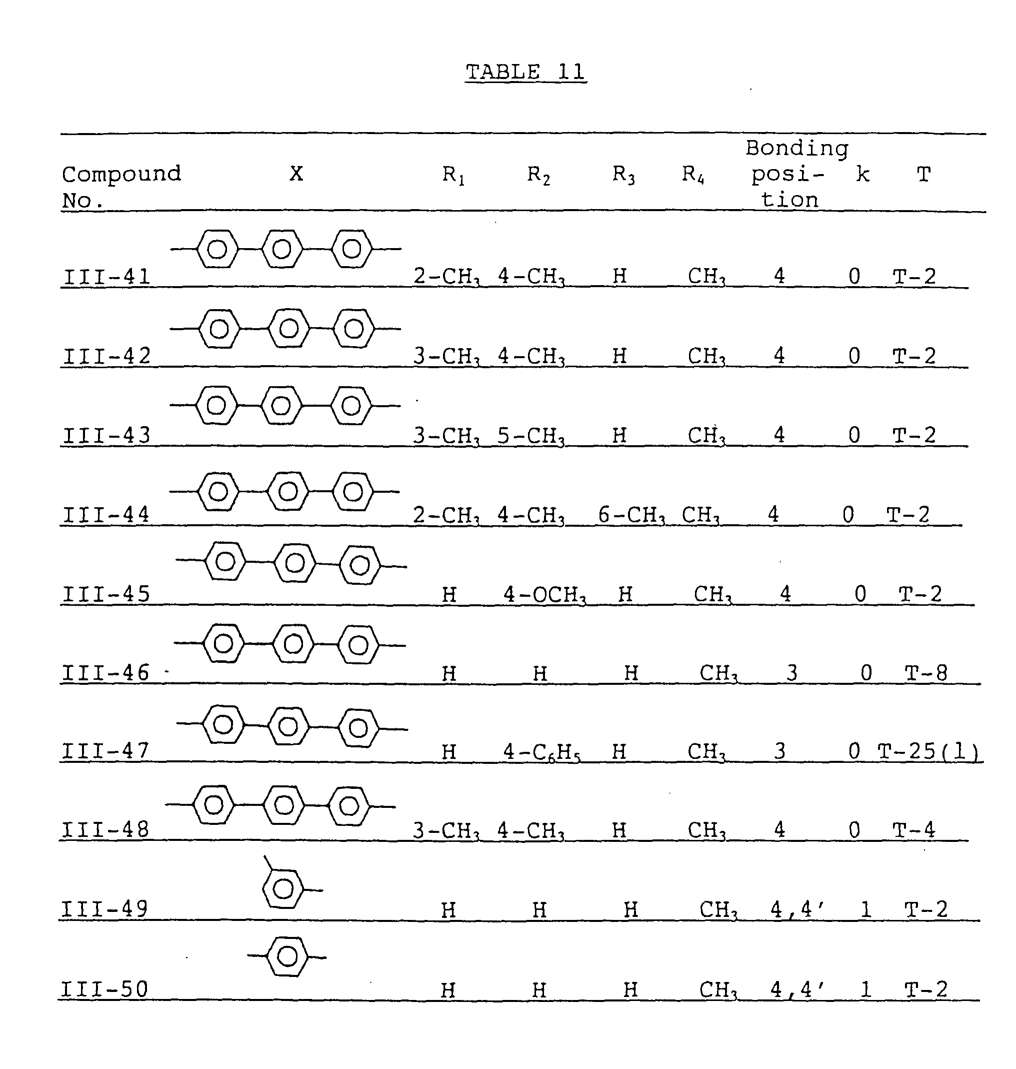

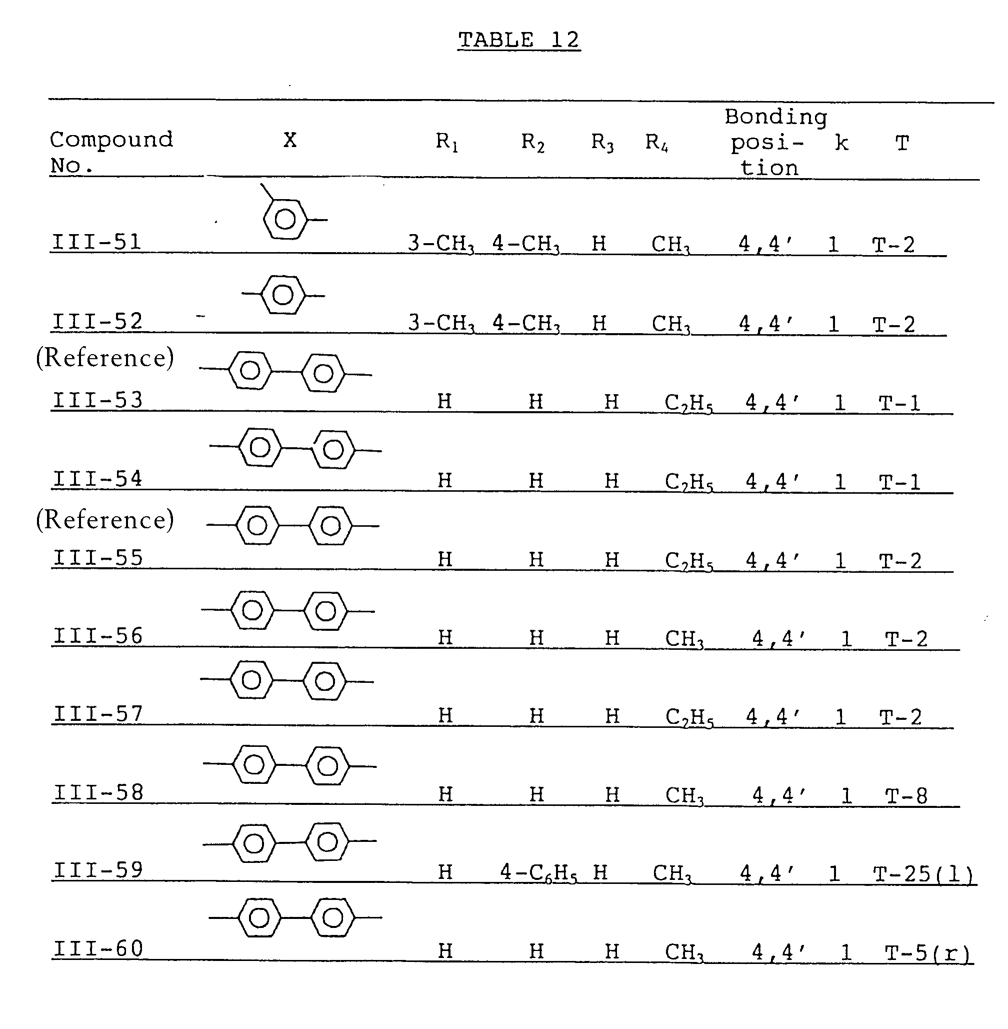

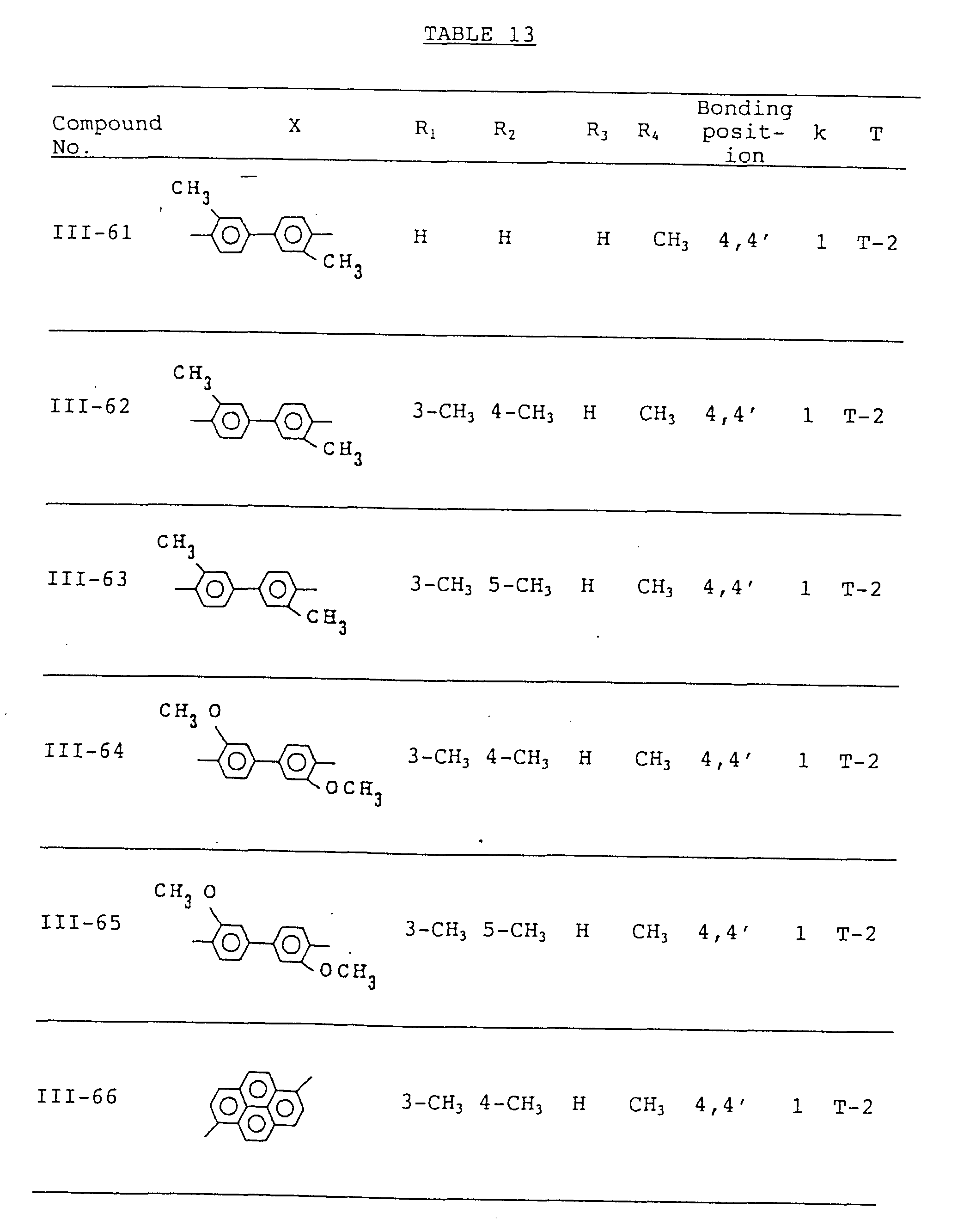

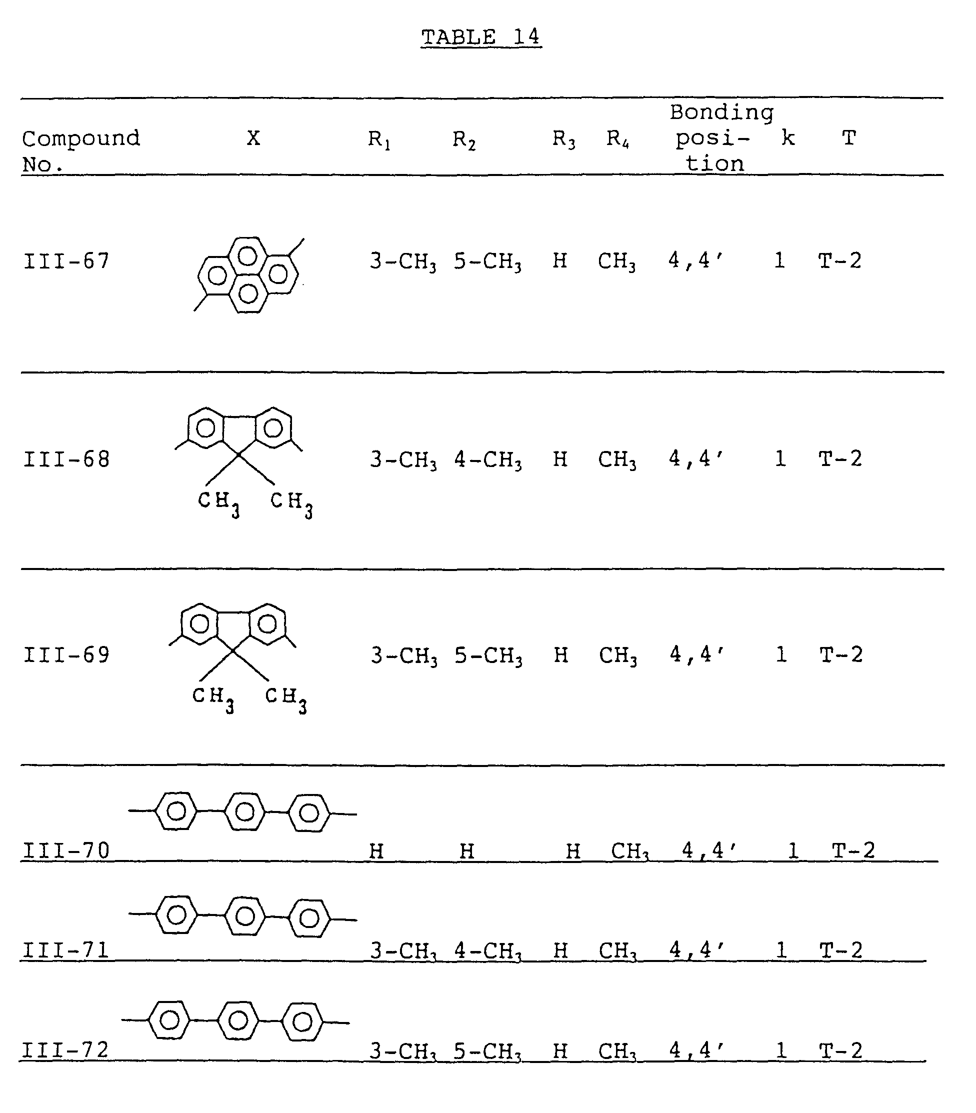

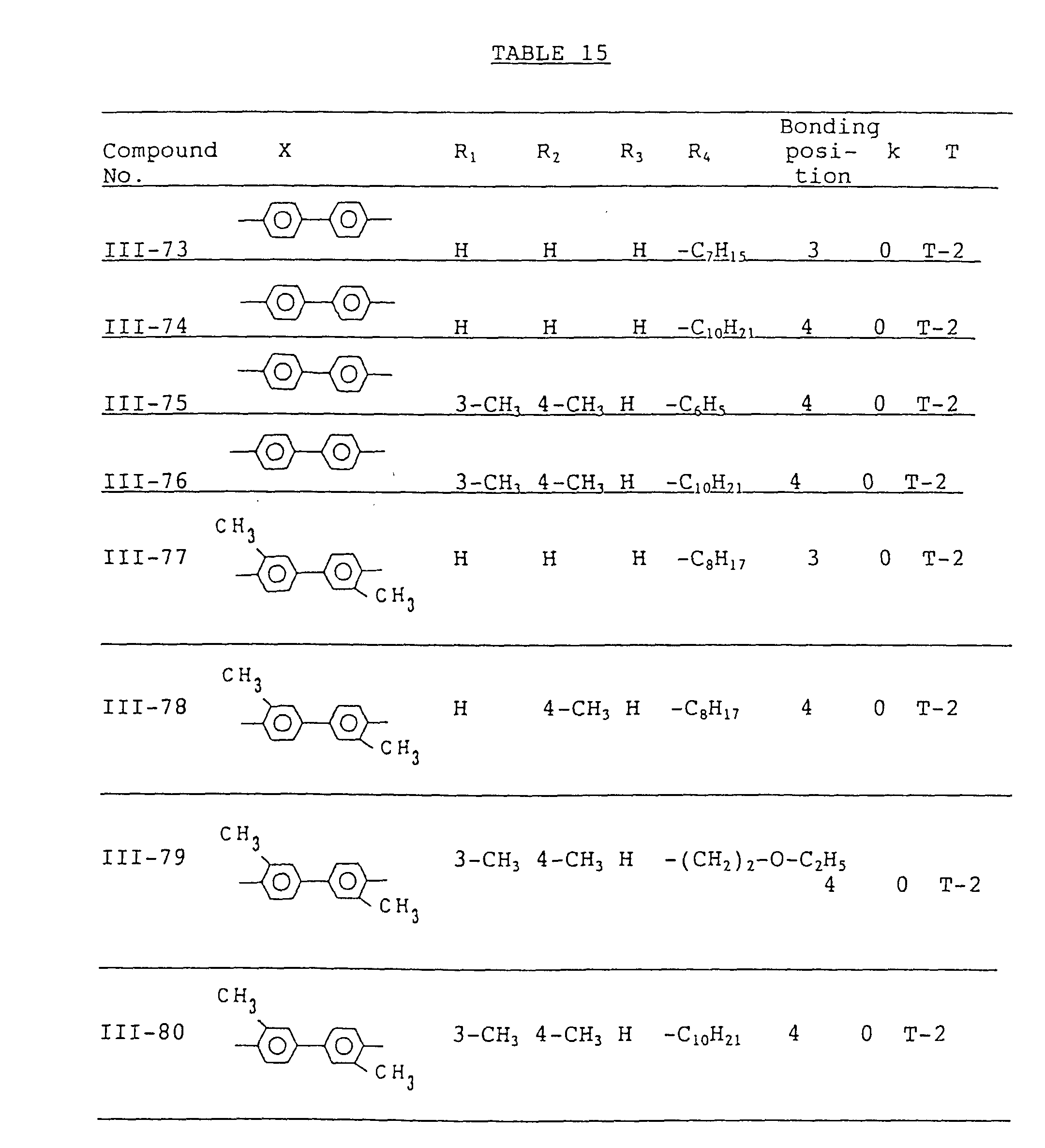

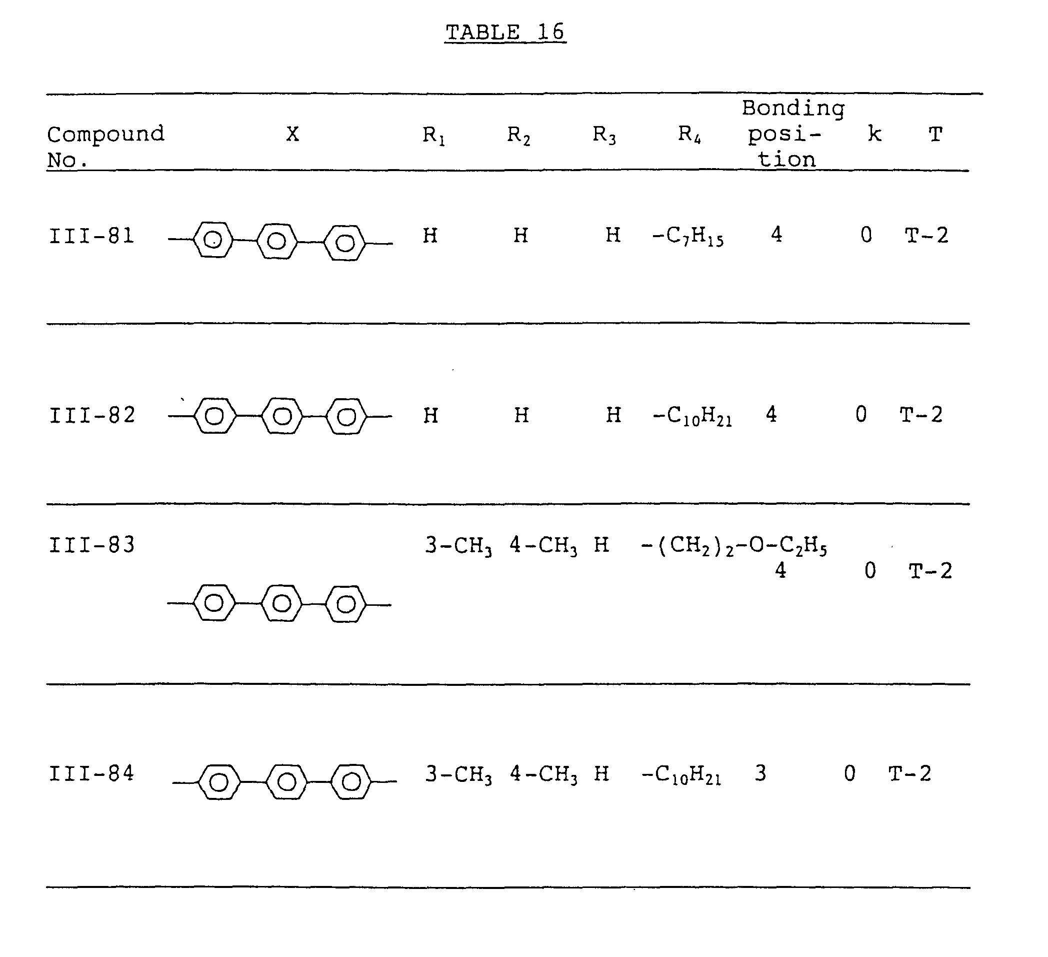

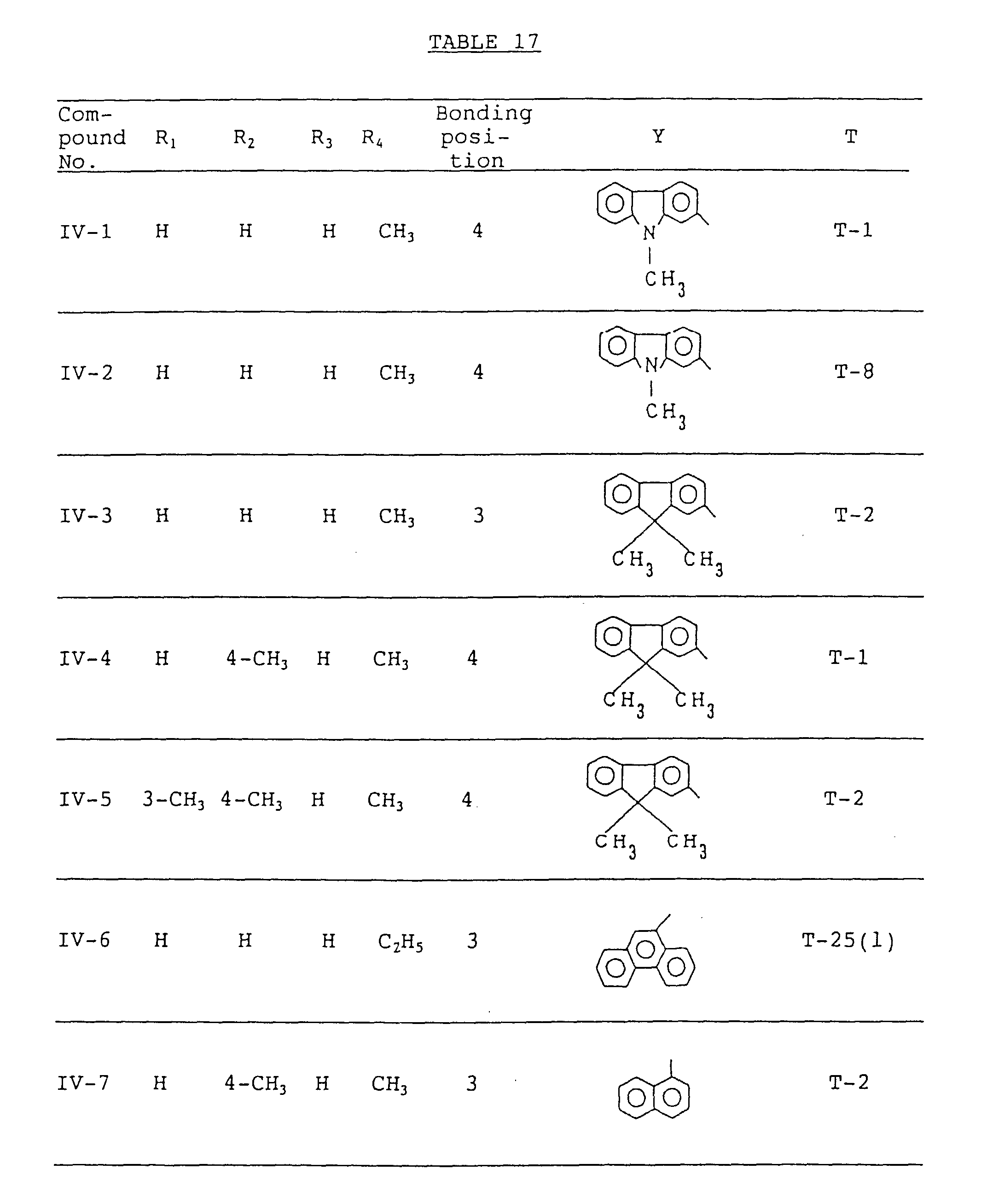

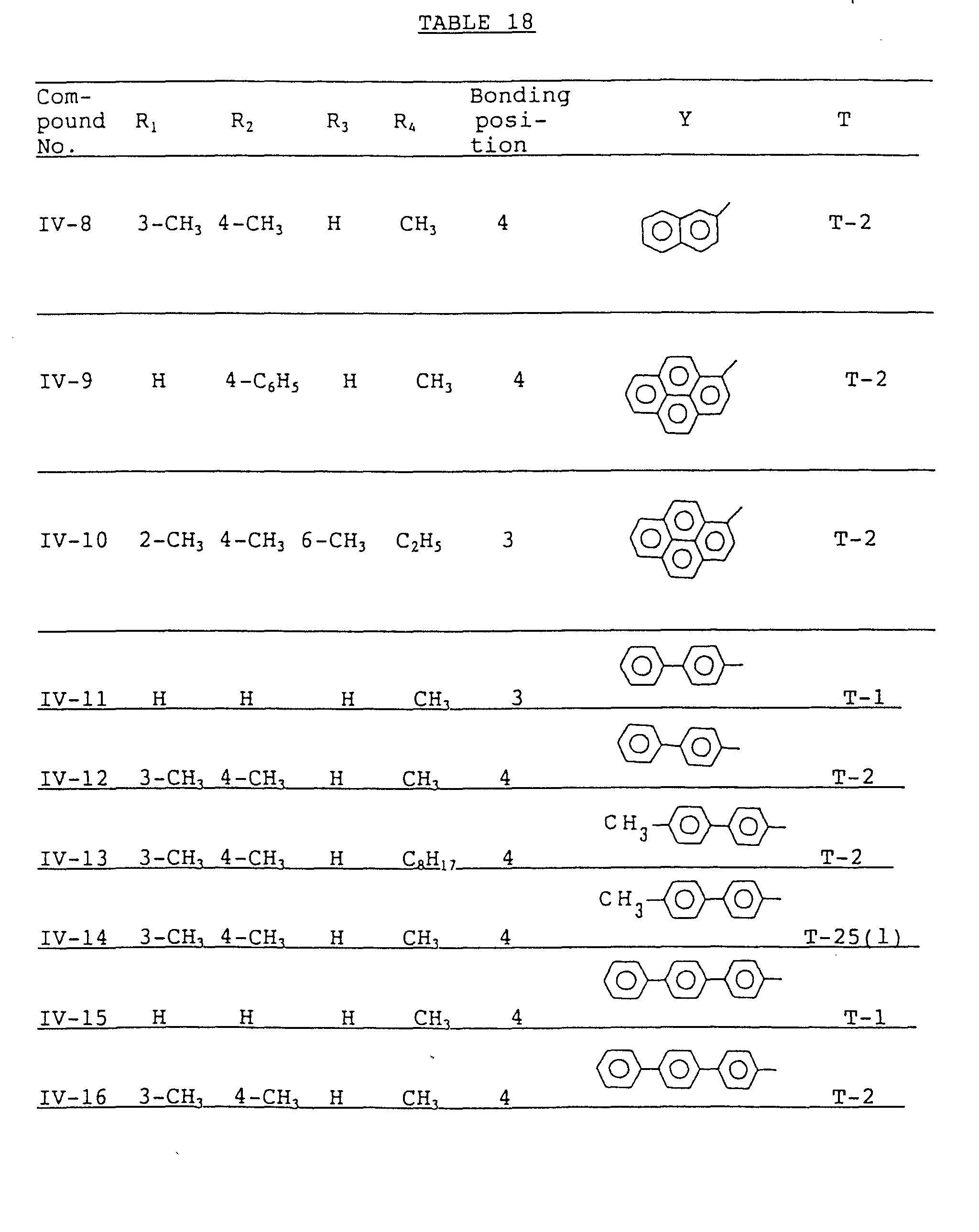

The present invention will be further described in the following examples hereinafter.

The compound represented by formula (I) to be used as a starting material from which the amine compound of the present invention is prepared can be synthesized as follows.

The compound No. in parentheses each indicates the compound having the chemical structure shown in the tables.

### SYNTHESIS EXAMPLE 1 (Synthesis of Compound (I-3))

Into a 1,000-mℓ flask were charged 60.0 g (0.44 mol) of phenylacetic acid, 500 mℓ of acetic acid, 60 mℓ of distilled water, 25 mℓ of concentrated sulfuric acid, 47.2 g (0.19 mol) of iodine, and 17.2 g (76 mmol) of periodic acid dihydrate. The reaction system was allowed to undergo reaction at a temperature of 105°C for 4 hours. After the iodine color of the reaction solution disappeared, the reaction solution was poured into 2 ℓ of distilled water. The reaction solution was then extracted with toluene. The resulting organic phase was thoroughly washed with dilute aqueous solution of sodium thiosulfate, dilute aqueous solution of sodium carbonate and distilled water, and then dried over anhydrous sodium sulfate. The solution was then concentrated to undergo crystallization. As a result, 41.4 g of Exemplary Compound (I-3) was obtained. The melting point of the compound thus obtained was from 142°C to 144°C.

### SYNTHESIS EXAMPLE 2 (Synthesis of Compound (I-6))

Into a 200-mℓ flask were charged 20 g of Compound (I-3) obtained in Synthesis Example 1, 80 mℓ of ethanol, and 1 mℓ of concentrated sulfuric acid. The mixture was then heated under reflux for 2 hours. The reaction solution was then poured into 2 ℓ of distilled water. The reaction solution was then extracted with toluene. The resulting organic phase was then thoroughly washed with dilute aqueous solution of sodium carbonate and distilled water. The reaction solution was then dried over anhydrous sodium sulfate. The solvent was then distilled off under reduced pressure to obtain 21.6 g of Compound (I-6) in the form of colorless oily matter.

### SYNTHESIS EXAMPLE 3 (Synthesis of Compound (I-9))

Into a 500-mℓ flask were charged 50.3 g (0.34 mol) of hydrocinnamic acid, 250 mℓ of acetic acid, 38 mℓ of distilled water, 19 mℓ of concentrated sulfuric acid, 36 g (0.14 mol) of iodine, and 13.2 g (57 mmol) of periodic acid dihydrate. The reaction system was allowed to undergo reaction at a temperature of 105°C for 4 hours. After the iodine color of the reaction solution disappeared, 50 mℓ of distilled water was poured into the reaction solution. The reaction solution was then allowed to cool to room temperature to undergo crystallization. The resulting precipitate was thoroughly washed with water, dried over anhydrous sodium sulfate, and then recrystallized from toluene to obtain 52.4 g of Compound (I-9). The melting point of the compound thus obtained was from 146°C to 148°C.

### SYNTHESIS EXAMPLE 4 (Synthesis of Compound (I-11))

Into a 200-mℓ flask was charged 100 mℓ of diethyl malonate. 2.6 g of metallic sodium was then added to the reaction system in an atmosphere of nitrogen. The reaction system was then stirred at room temperature until the metallic sodium was all dissolved. Thereafter, 33.1 g (112 mmol) of 3-iodobenzyl bromide was gradually added dropwise to the reaction system. After the completion of the dropwise addition, the reaction system was allowed to further undergo reaction at a temperature of about 80°C. After the completion of the reaction, the reaction system was allowed to cool to room temperature. To the reaction system was then added 100 mℓ of toluene. The resulting salt was then filtered off. The filtrate was then concentrated. To the filtrate were then added 9.5 g (223 mmol) of lithium chloride, 2 mℓ of distilled water, and 60 mℓ of dimethyl sulfoxide. The reaction mixture was heated under reflux for 2 hours, poured into 1 ℓ of distilled water, and then extracted with toluene. The resulting organic phase was thoroughly washed with distilled water, and then dried over anhydrous sodium sulfate. The solvent was then distilled off under reduced pressure. The residue was then subjected to distillation under reduced pressure to obtain 23.0 g of Compound (I-11) in the form of colorless oily matter. The boiling point of the compound thus obtained was 140°C at 0.5 to 0.6 mmHg.

### SYNTHESIS EXAMPLE 5 (Synthesis of Compound (I-12))

20 g of Compound (I-9) obtained in Synthesis Example 3 was subjected to ethyl-esterification in the same manner as in Synthesis Example 2. The ethyl-esterification product was then subjected to distillation under reduced pressure to obtain 21.0 g of Compound (I-12) in the form of colorless oily matter. The boiling point of the compound thus obtained was 105°C at 0.1 to 0.15 mmHg.

### SYNTHESIS EXAMPLE 6 (Synthesis of Compound (I-18))

Into a 300-mℓ flask were charged 25.1 g (153 mmol) of 4-phenyl-n-butyric acid, 120 mℓ of acetic acid, 15 mℓ of distilled water, 9 mℓ of concentrated sulfuric acid, 16.4 g (64 mol) of iodine, and 6 g (26 mmol) of periodic acid dihydrate. The reaction mixture was then allowed to undergo reaction at a temperature of 105°C for 4 hours. After the iodine color of the reaction solution disappeared, 100 mℓ of distilled water was added to the reaction solution. The reaction solution was then allowed to cool to room temperature so that crystallization occurred. The resulting crystal was thoroughly washed with water, dried, and then recrystallized from a mixture of toluene and hexane to obtain 20.5 g of Compound (I-18). The melting point of the compound thus obtained was from 95°C to 96°C.

### SYNTHESIS EXAMPLE 7 (Synthesis of Compound (I-30))

Into a 300-mℓ flask were charged 25.2 g (119 mol) of biphenylacetic acid, 150 mℓ of acetic acid, 16 mℓ of distilled water, 7 mℓ of concentrated sulfuric acid, 12.7 g (50 mmol) of iodine, and 4.7 g (20 mmol) of periodic acid dihydrate. The reaction system was allowed to undergo reaction at a temperature of 105°C for 4 hours. After the iodine color of the reaction solution disappeared, 100 mℓ of distilled water was added to the reaction solution. The reaction solution was then allowed to cool to room temperature so that crystallization occurred. The resulting crystal was thoroughly washed with water. The crystal was then charged into a 500-mℓ flask. Into the flask was then charged 200 mℓ of toluene. The reaction system was then heated under reflux so that water was boiled away azeotropically with toluene. Into the flask were then charged 200 mℓ of ethanol and 2 mℓ of concentrated sulfuric acid. The reaction mixture was then heated under reflux for 2 hours. The reaction system was treated in the same manner as in Synthesis Example 2, and then subjected to distillation under reduced pressure to obtain 28.7 g of Compound (I-30) in the form of colorless oily matter. The boiling point of the compound thus obtained was 185°C at 0.2 to 0.3 mmHg.

### SYNTHESIS EXAMPLE 8 (Synthesis of Compound (I-34))

Into a 1,000 mℓ flask were charged 100 g (0.36 mol) of 4-iodobiphenyl, 13.9 g (0.46 mol) of paraformaldehyde, 55.1 g (0.54 mol) of sodium bromide, and 50 mℓ of acetic acid. The reaction mixture was heated to a temperature of 100°C where a mixture of 45 mℓ of acetic acid and 45 mℓ of concentrated sulfuric acid was then gradually added thereto. The reaction system was heated under reflux for 24 hours, poured into 500 mℓ of distilled water, and then extracted with methylene chloride. The resulting organic phase was thoroughly washed with distilled water, and then dried over anhydrous sodium sulfate. The solvent was distilled off under reduced pressure. The residue was then recrystallized from a mixture of ethyl acetate and ethanol to obtain 43.6 g of 4-bromomethyl-4'-iodobiphenyl. 40 g of 4-bromomethyl-4'-iodobiphenyl thus obtained was then allowed to undergo reaction in the same manner as in Synthesis Example 4. The reaction product was treated in the same manner as in Synthesis Example 4, and then subjected to distillation under reduced pressure to obtain 20.4 g of Compound (I-34) in the form of colorless oily matter. The boiling point of the compound thus obtained was 190°C at 0.1 to 0.15 mmHg.

### EXAMPLE 1 (Synthesis of II-8)

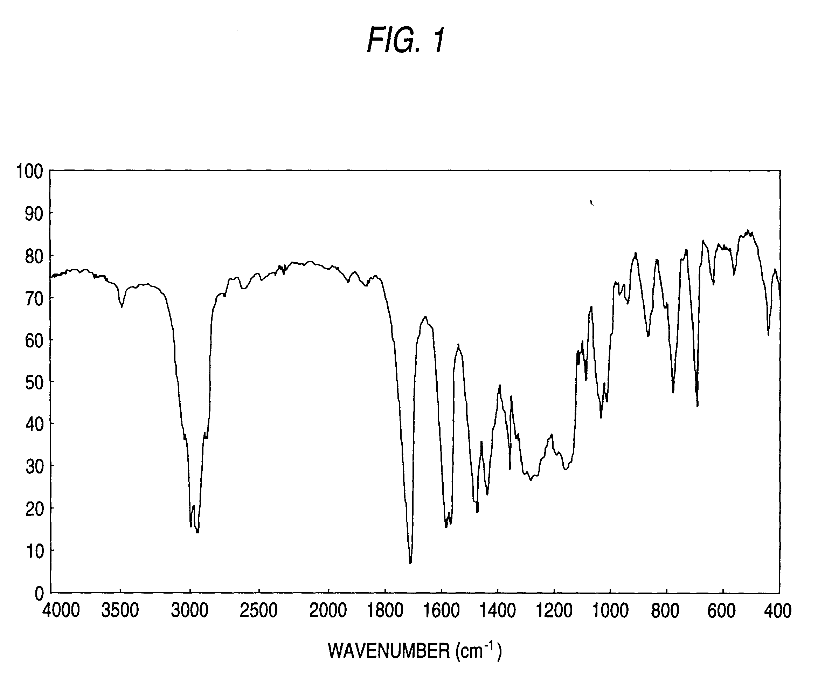

Into a 500-mℓ flask were charged 6.0 g of 3,4-xylidine, 34.0 g of Compound (I-11), 19.0 g of potassium carbonate, 2.0 g of copper sulfate pentahydrate, and 50 mℓ of n-tridecane. The reaction mixture was then allowed to undergo reaction at an elevated temperature of 230°C in a stream of nitrogen for 10 hours. After the completion of the reaction, the reaction system was allowed to cool to room temperature. The reaction system was then dissolved in 200 mℓ of toluene. The insoluble matters were then filtered off. The filtrate was then purified by silica gel column chromatography with toluene to obtain 20.3 g of Compound (II-8) in the form of oily matter. IR spectrum of Compound (II-8) thus obtained is shown in Fig. 1.

### EXAMPLE 2 (Synthesis of II-23)

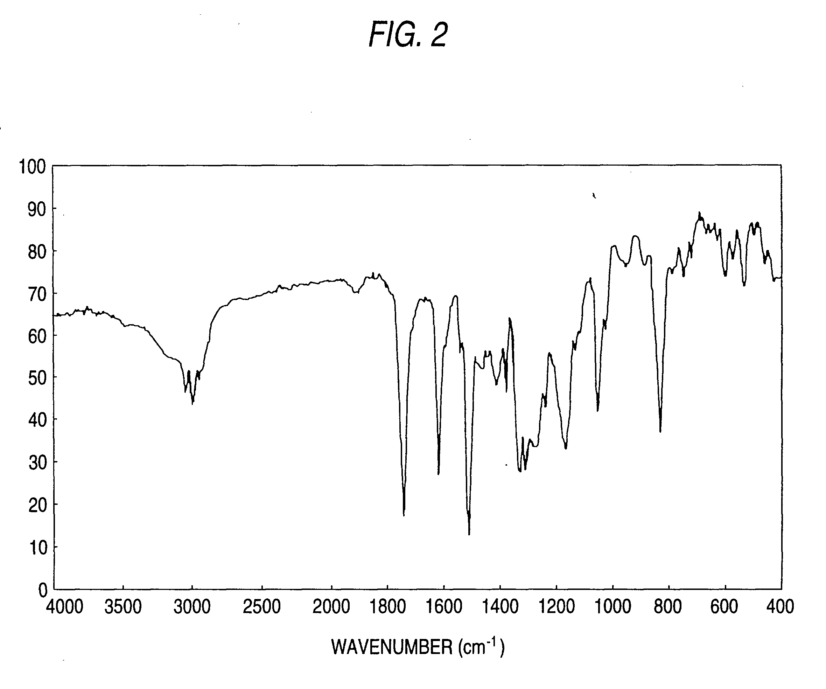

Into a 500-mℓ flask were charged 10.0 g of 3,4-xylidine, 65.0 g of Compound (I-30), 30.0 g of potassium carbonate, 1.0 g of copper sulfate pentahydrate, and 50 mℓ of n-tridecane. The reaction mixture was then allowed to undergo reaction at an elevated temperature of 230°C in a stream of nitrogen for 8 hours. After the completion of the reaction, the reaction system was allowed to cool to room temperature. The reaction system was then dissolved in 100 mℓ of toluene. The insoluble matters were then filtered off. The filtrate was then purified by silica gel column chromatography with toluene to obtain 28.5 g of Compound (II-23) in the form of oily matter. IR spectrum of Compound (II-23) thus obtained is shown in Fig. 2.

### EXAMPLE 3 (Synthesis of III-6)

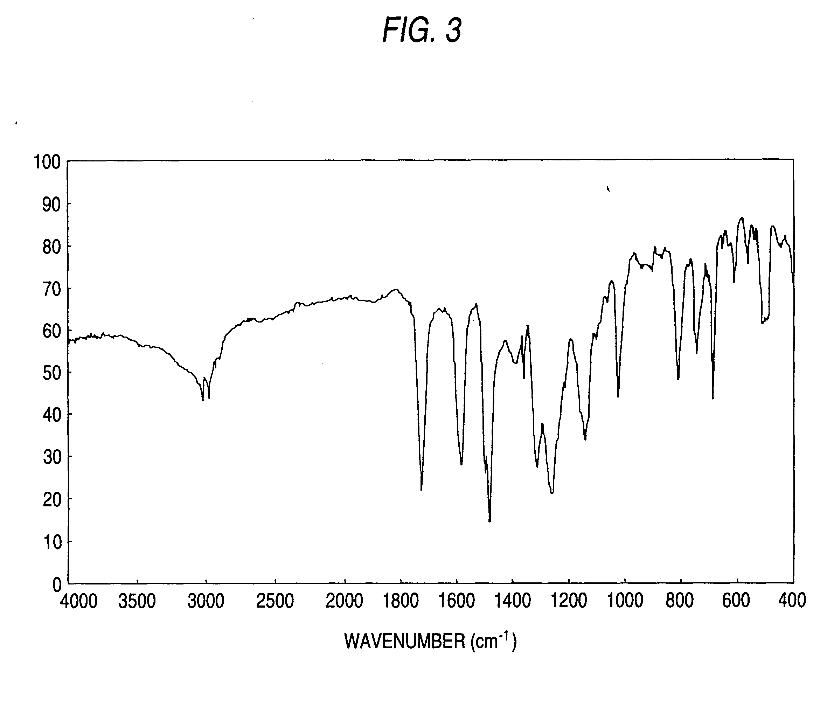

Into a 100-mℓ flask were charged 5.3 g of N, N'-diphenylbenzidine, 10.0 g of Compound (I-6), 5.6 g of potassium carbonate, 0.5 g of copper sulfate pentahydrate, and 10 mℓ of n-tridecane. The reaction mixture was then allowed to undergo reaction at an elevated temperature of 230°C in a stream of nitrogen for 1 hour. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene to obtain 6.2 g of Compound (III-6) in the form of oily matter. IR spectrum of Compound (III-6) thus obtained is shown in Fig. 3.

### REFERENCE EXAMPLE 4 (Synthesis of III-7)

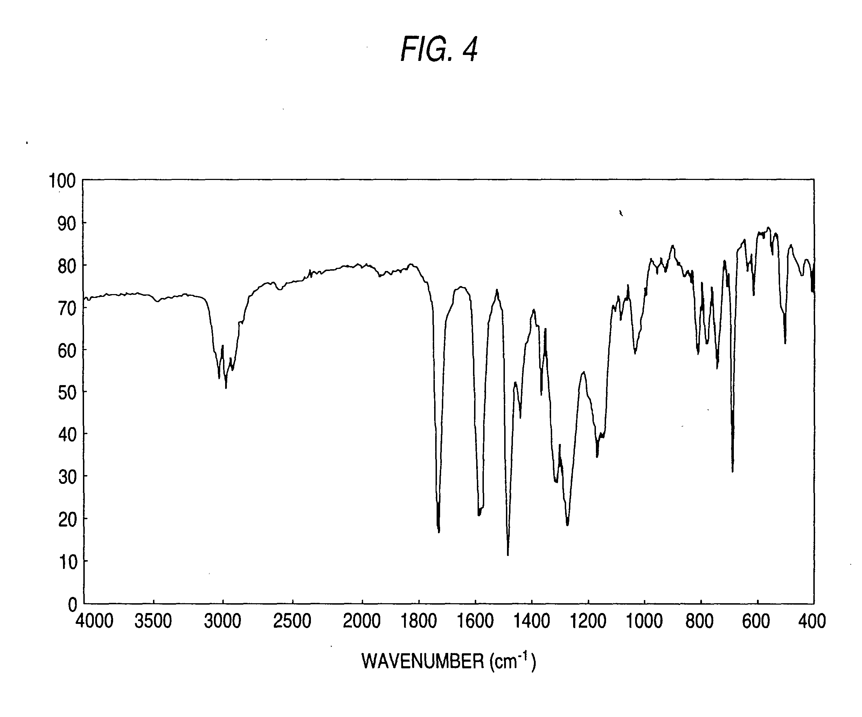

Into a 100-mℓ flask were charged 10.8 g of N, N'-diphenylbenzidine, 23.0 g of Compound (I-11), 11.6 g of potassium carbonate, 1.0 g of copper sulfate pentahydrate, and 20 mℓ of n-tridecane. The reaction mixture was then allowed to undergo reaction at an elevated temperature of 230°C in a stream of nitrogen for 1 hour. After the completion of the reaction, the reaction system was allowed to cool to room temperature. The reaction system was then dissolved in 50 mℓ of toluene. The insoluble matters were then filtered off. The filtrate was then purified by silica gel column chromatography with toluene to obtain 19.6 g of Compound (III-7) in the form of oily matter. IR spectrum of Compound (III-7) thus obtained is shown in Fig. 4.

### EXAMPLE 5 (Synthesis of III-9)

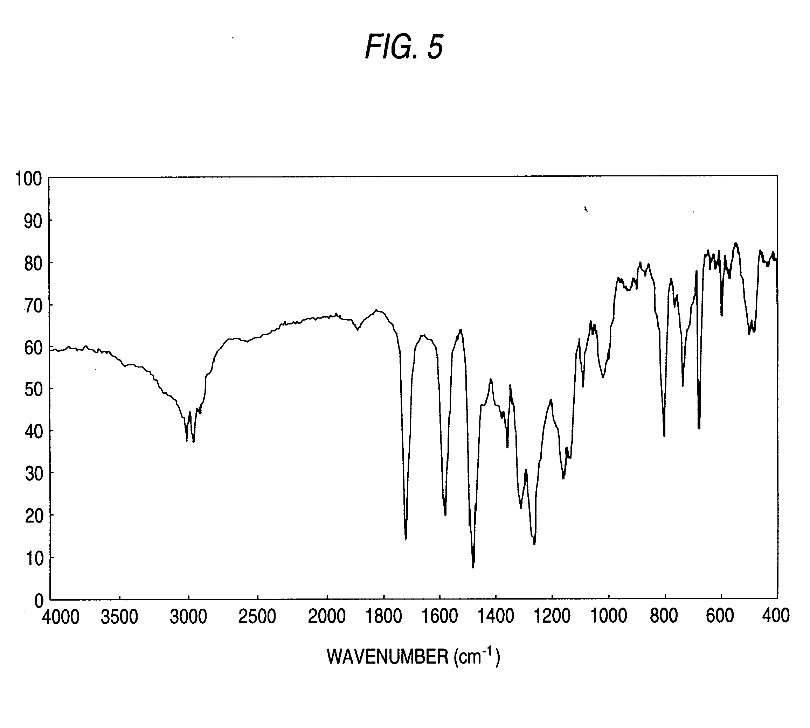

Into a 100-mℓ flask were charged 10.1 g of N, N'-diphenylbenzidine, 20.0 g of Compound (I-12), 10.9 g of potassium carbonate, 1.0 g of copper sulfate pentahydrate, and 20 mℓ of n-tridecane. The reaction mixture was then allowed to undergo reaction at an elevated temperature of 230°C in a stream of nitrogen for 1.5 hours. After the completion of the reaction, the reaction system was allowed to cool to room temperature. The reaction system was then dissolved in 50 mℓ of toluene. The insoluble matters were then filtered off. The filtrate was then purified by silica gel column chromatography with toluene to obtain 18.7 g of Compound (III-9) in the form of oily matter. IR spectrum of Compound (III-9) thus obtained is shown in Fig. 5.

### EXAMPLE 6 (Synthesis of III-14)

Into a 200-mℓ flask were charged 16.3 g of 3,4-dimethylacetanilide, 24.8 g of Compound (I-3), 15.5 g of potassium carbonate, 10.0 g of copper powder, and 10 mℓ of n-tridecane. The reaction mixture was the allowed to undergo reaction at an elevated temperature of 200°C for 24 hours. After the completion of the reaction, 60 mℓ of ethylene glycol and 10.0 g of potassium hydroxide were added to the reaction system. The reaction system was then heated under reflux in a stream of nitrogen for 2 hours. The reaction system was allowed to cool to room temperature, poured into 1 ℓ of water, and then neutralized with hydrochloric acid to undergo crystallization. The crystal was filtered off. The filtrate was thoroughly washed with water, and then transferred to a 500-mℓ flask. Into the flask was then charged 200 mℓ of toluene. The reaction system was then heated under reflux so that water was azeotropically boiled away. To the reaction system were then added 100 mℓ of methanol and 3 mℓ of concentrated sulfuric acid. The reaction system was then heated under reflux in a stream of nitrogen for 2 hours. After the completion of the reaction, the reaction solution was poured into 2 ℓ of distilled water, and then extracted with toluene. The resulting organic phase was thoroughly washed with dilute aqueous solution of sodium carbonate and distilled water, and then dried over anhydrous sodium sulfate. The solvent was then distilled off under reduced pressure. The residue was then recrystallized from methanol to obtain 21.3 g of N-(3,4-dimethylphenyl)-N-[4-(methoxycarbonylmethyl)phenyl]amine.

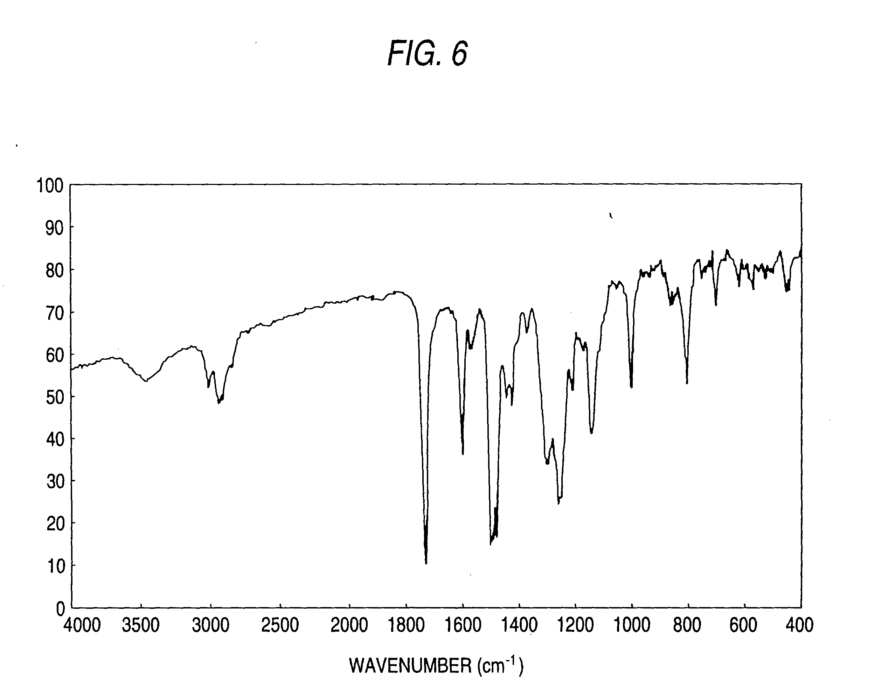

Into a 200-mℓ flask were charged 9.0 g of N-(3,4-dimethylphenyl)-N-[4-(methoxycarbonylmethyl)phenyl]amine thus obtained, 6.2 g of 4,4'-diiodo-3,3'-dimethylbiphenyl, 5.5 g of potassium carbonate, 1.0 g of copper sulfate pentahydrate, and 10 mℓ of n-tridecane. The reaction mixture was then allowed to undergo reaction at an elevated temperature of 230°C in a stream of nitrogen for 5 hours. After the completion of the reaction, the reaction system was allowed to cool to room temperature. The reaction system was then dissolved in 40 mℓ of toluene. The insoluble matters were then filtered off. The filtrate was then purified by silica gel column chromatography with toluene. The material was then recrystallized from a mixture of ethyl acetate and ethanol to obtain 7.1 g of Compound (III-14) in the form of light yellow powder. The melting point of Compound (III-14) thus obtained was from 179°C to 181°C. IR spectrum of the compound thus obtained is shown in Fig. 6.

### EXAMPLE 7 (Synthesis of III-16)

Into a 200-mℓ flask were charged 6.7 g of p-acetotoluidide, 10.8 g of Compound (I-9), 5.7 g of potassium carbonate, 1.0 g of copper sulfate pentahydrate, and 20 mℓ of n-tridecane. The reaction mixture was the allowed to undergo reaction at an elevated temperature of 230°C for 3.5 hours. After the completion of the reaction, 33 mℓ of ethylene glycol and 6.6 g of potassium hydroxide were added to the reaction system. The reaction system was then heated under reflux in a stream of nitrogen for 2 hours. The reaction system was allowed to cool to room temperature, poured into 50 mℓ of water, and then neutralized with hydrochloric acid to undergo crystallization. The crystal was withdrawn by filtration, thoroughly washed with water, and then transferred to a 500-mℓ flask. Into the flask was then charged 100 mℓ of toluene. The reaction system was then heated under reflux so that water was azeotropically boiled away. To the reaction system were then added 50 mℓ of methanol and 1 mℓ of concentrated sulfuric acid. The reaction system was then heated under reflux in a stream of nitrogen for 1 hour. After the completion of the reaction, the reaction solution was poured into 200 mℓ of distilled water, and then extracted with toluene. The resulting organic phase was then thoroughly washed with dilute aqueous solution of sodium carbonate and distilled water. To the organic phase was then added 10 g of active clay. The mixture was then heated under reflux for 1 hour. The active clay used was filtered off. The filtrate was then concentrated to obtain 6.7 g of N-(4-methylphenyl)-N-[4-(2-methoxycarbonylethyl)phenyl]amine.

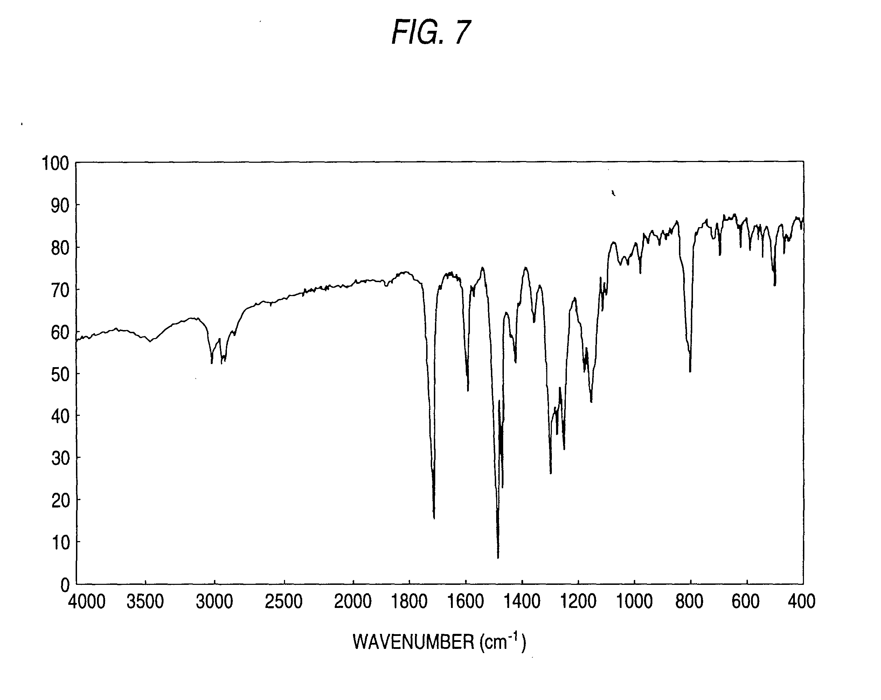

Into a 100-mℓ flask were charged 6.0 g of N-(4-methylphenyl)-N-[4-(2-methoxycarbonylethyl)phenyl]amine thus obtained, 4.3 g of 4,4'-diiodo-3,3'-dimethylbiphenyl, 1.6 g of potassium carbonate, 0.5 g of copper sulfate pentahydrate, and 10 mℓ of n-tridecane. The reaction mixture was then allowed to undergo reaction at an elevated temperature of 230°C in a stream of nitrogen for 5 hours. After the completion of the reaction, the reaction system was allowed to cool to room temperature. The reaction system was then dissolved in 20 mℓ of toluene. The insoluble matters were then filtered off. The filtrate was then purified by active clay with toluene. The material was then recrystallized from acetone to obtain 5.6 g of Compound (III-16) in the form of white powder. The melting point of Compound (III-16) thus obtained was from 183°C to 184°C. IR spectrum of the compound thus obtained is shown in Fig. 7.

### EXAMPLE 8 (Synthesis of III-18)

Into a 200-mℓ flask were charged 29.3 g of 3,4-dimethylacetanilide, 40.0 g of Compound (I-9), 22.3 g of potassium carbonate, 1.0 g of copper sulfate pentahydrate, and 200 mℓ of n-tridecane. The reaction mixture was then allowed to undergo reaction at an elevated temperature of 230°C for 5 hours. After the completion of the reaction, 100 mℓ of ethylene glycol and 26.0 g of potassium hydroxide were added to the reaction system. The reaction system was then heated under reflux in a stream of nitrogen for 2 hours. The reaction system was allowed to cool to room temperature, poured into 1 ℓ of water, and then neutralized with hydrochloric acid to undergo crystallization. The crystal was withdrawn by filtration, thoroughly washed with water, and then transferred to a 500-mℓ flask. Into the flask was then charged 200 mℓ of toluene. The reaction system was then heated under reflux so that water was azeotropically boiled away. To the reaction system were then added 100 mℓ of methanol and 3 mℓ of concentrated sulfuric acid. The reaction system was then heated under reflux in a stream of nitrogen for 2 hours. After the completion of the reaction, the reaction solution was poured into 2 ℓ of distilled water, and then extracted with toluene. The resulting organic phase was thoroughly washed with dilute aqueous solution of sodium carbonate and distilled water, and then dried over anhydrous sodium sulfate. The solvent was then distilled off under reduced pressure. The residue was then recrystallized from methanol to obtain 33.2 g of N-(3,4-dimethylphenyl)-N-[4-(2-methoxycarbonylethyl)phenyl]amine.

Into a 500-mℓ flask were charged 4.5 g of N-(3,4-dimethylphenyl)-N-[4-(2-methoxycarbonylethyl)phenyl]amine thus obtained, 3.0 g of 4,4'-diiodo-3,3'-dimethylbiphenyl, 2.7 g of potassium carbonate, 0.5 g of copper sulfate pentahydrate, and 5 mℓ of n-tridecane. The reaction mixture was then allowed to undergo reaction at an elevated temperature of 230°C in a stream of nitrogen for 5 hours. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene. The material was then recrystallized from a mixture of ethyl acetate and ethanol to obtain 3.8 g of Compound (III-18) in the form of light yellow powder.

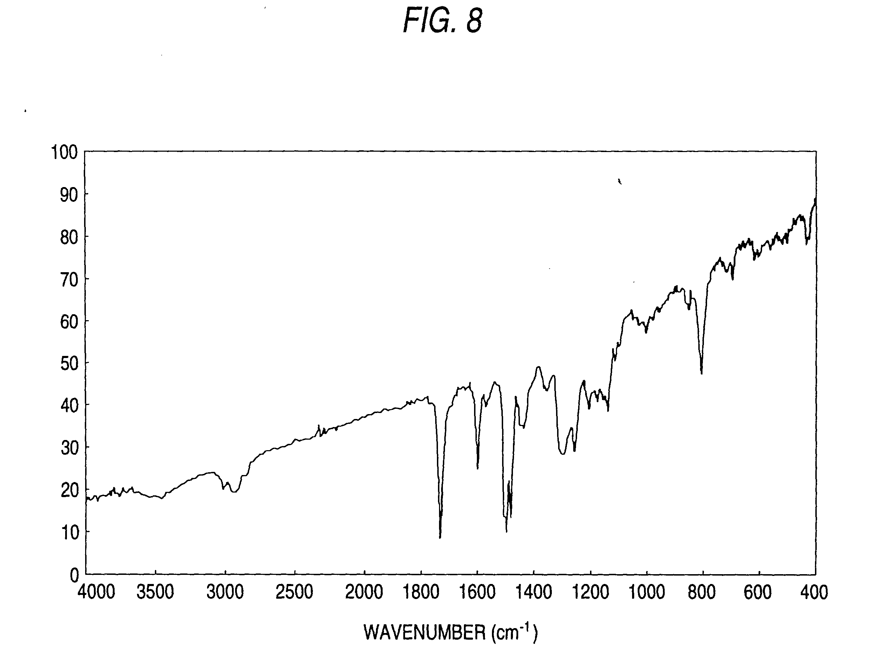

The melting point of Compound (III-18) thus obtained was from 162°C to 163°C. IR spectrum of the compound thus obtained is shown in Fig. 8.

### REFERENCE EXAMPLE 1

In the hydrolyzation step of Example 8, ethanol was used instead of ethylene glycol. As a result, the reaction product was not dissolved. Thus, hydrolyzation required 48 hours. Thus, it was found that the use of ethanol requires prolonged hydrolyzation as compared with the use of ethylene glycol.

### EXAMPLE 9 (Synthesis of III-19)

Into a 500-mℓ flask were charged 30.0 g of 3,4-dimethylacetanilide, 42.0 g of Compound (I-9), 23.2 g of potassium carbonate, 1.0 g of copper sulfate pentahydrate, and 50 mℓ of n-tridecane. The reaction mixture was then allowed to undergo reaction at an elevated temperature of 230°C for 5 hours. After the completion of the reaction, 100 mℓ of ethylene glycol and 25.0 g of potassium hydroxide were added to the reaction system. The reaction system was then heated under reflux in a stream of nitrogen for 2 hours. The reaction system was allowed to cool to room temperature, poured into 500 mℓ of water, and then neutralized with hydrochloric acid to undergo crystallization. The crystal was withdrawn by filtration, thoroughly washed with water, and then transferred to a 500-mℓ flask. Into the flask was then charged 200 mℓ of toluene. The reaction system was then heated under reflux so that water was azeotropically boiled away. To the reaction system were then added 100 mℓ of methanol and 2 mℓ of concentrated sulfuric acid. The reaction system was then heated under reflux in a stream of nitrogen for 2 hours. After the completion of the reaction, the reaction solution was poured into 300 mℓ of distilled water, and then extracted with toluene. The resulting organic phase was thoroughly washed with dilute aqueous solution of sodium carbonate and distilled water, and then dried over anhydrous sodium sulfate. The solvent was then distilled off under reduced pressure. The residue was then recrystallized from a mixture of toluene and hexane to obtain 35.5 g of N-(3,5-dimethylphenyl)-N-[4-(2-methoxycarbonyl ethyl)phenyl]amine.

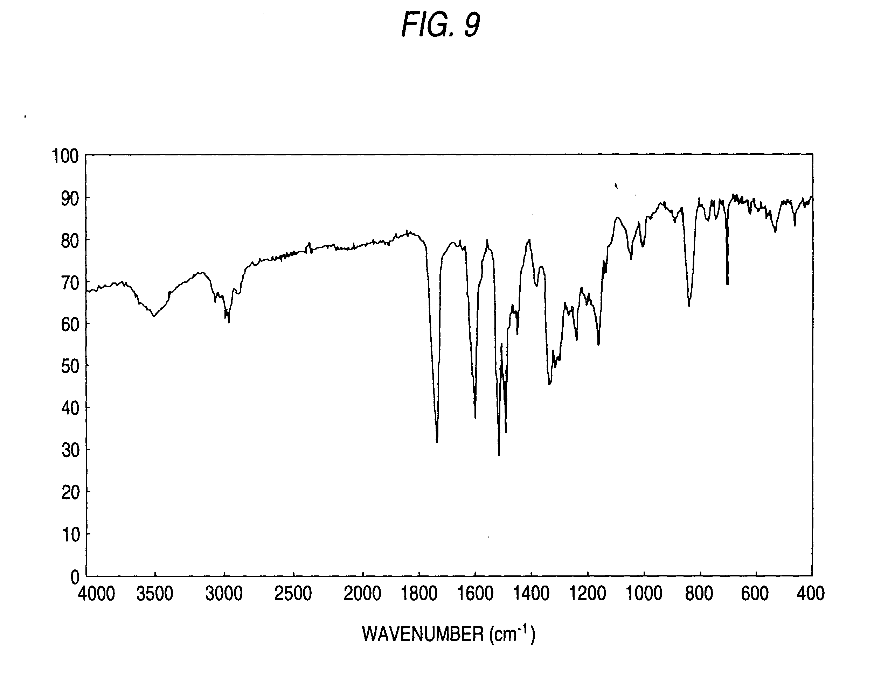

Into a 500-mℓ flask were charged 15.0 g of N-(3,5-dimethylphenyl)-N-[4-(2-methoxycarbonylethyl)phenyl]amine thus obtained, 14.0 g of 4,4'-diiodo-3,3'-dimethylbiphenyl, 8.0 g of potassium carbonate, 0.5 g of copper sulfate pentahydrate, and 20 mℓ of n-tridecane. The reaction mixture was then allowed to undergo reaction at an elevated temperature of 230°C in a stream of nitrogen for 3 hours. After the completion of the reaction, the reaction system was allowed to cool to room temperature. The reaction system was then dissolved in 100 mℓ of toluene. The insoluble matters were then filtered off. The filtrate was then purified by silica gel column chromatography with toluene. The material was then recrystallized from a mixture of ethyl acetate and ethanol to obtain 16.0 g of Compound (III-19) in the form of light yellow powder. The melting point of Compound (III-19) thus obtained was from 177°C to 179°C. IR spectrum of the compound thus obtained is shown in Fig. 9.

### EXAMPLE 10 (Synthesis of III-21)

Into a 200-mℓ flask were charged 25.0 g of p-acetanisizide, 33.8 g of Compound (I-9), 19.2 g of potassium carbonate, 1.0 g of copper sulfate pentahydrate, and 15 mℓ of n-tridecane. The reaction mixture was then allowed to undergo reaction at an elevated temperature of 230°C for 5 hours. After the completion of the reaction, 100 mℓ of ethylene glycol and 22.0 g of potassium hydroxide were added to the reaction system. The reaction system was then heated under reflux in a stream of nitrogen for 2 hours. The reaction system was allowed to cool to room temperature, poured into 200 mℓ of water, and then neutralized with hydrochloric acid to undergo crystallization. The crystal was withdrawn by filtration, thoroughly washed with water, and then transferred to a 500-mℓ flask. Into the flask was then charged 200 mℓ of toluene. The reaction system was then heated under reflux so that water was azeotropically boiled away. To the reaction system were then added 100 mℓ of methanol and 3 mℓ of concentrated sulfuric acid. The reaction system was then heated under reflux in a stream of nitrogen for 2 hours. After the completion of the reaction, the reaction solution was poured into 2 ℓ of distilled water, and then extracted with toluene. The resulting organic phase was thoroughly washed with dilute aqueous solution of sodium carbonate and distilled water, and then dried over anhydrous sodium sulfate. The solvent was then distilled off under reduced pressure. The residue was then recrystallized from methanol to obtain 19.5 g of N-(4-methoxyphenyl)-N-[4-(2-methoxycarbonylethyl)phenyl]amine.

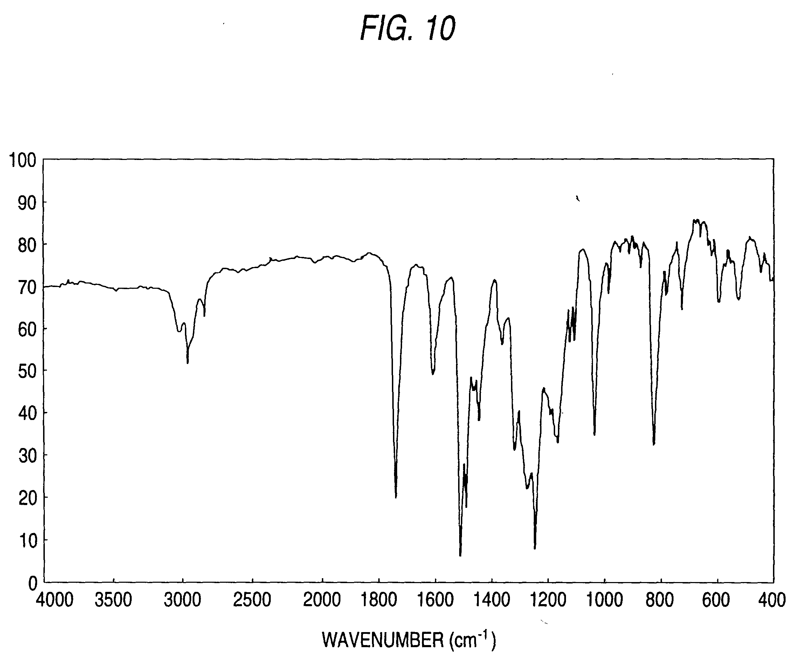

Into a 100-mℓ flask were charged 5.0 g of N-(4-methoxyphenyl)-N-[4-(2-methoxycarbonylethyl)phenyl]amine thus obtained, 3.4 g of 4,4'-diiodo-3,3'-dimethylbiphenyl, 2.9 g of potassium carbonate, 0.5 g of copper sulfate pentahydrate, and 5 mℓ of n-tridecane. The reaction mixture was then allowed to undergo reaction at an elevated temperature of 230°C in a stream of nitrogen for 15 hours. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene to obtain 5.3 g of Compound (III-21) in the form of light yellow oil. IR spectrum of Compound (III-21) thus obtained is shown in Fig. 10.

### EXAMPLE 11 (Synthesis of III-24)

Into a 300-mℓ flask were charged 15.6 g of 3,4-dimethylacetanilide, 19.0 g of Compound (I-18), 15.8 g of potassium carbonate, 1.0 g of copper sulfate pentahydrate, and 10 mℓ of n-tridecane. The reaction mixture was then allowed to undergo reaction at an elevated temperature of 210°C for 4 hours. After the completion of the reaction, 100 mℓ of ethylene glycol and 16.1 g of potassium hydroxide were added to the reaction system. The reaction system was then heated under reflux in a stream of nitrogen for 2 hours. The reaction system was allowed to cool to room temperature, poured into 1 ℓ of water, and then neutralized with hydrochloric acid to undergo crystallization. The crystal was withdrawn by filtration, thoroughly washed with water, and then transferred to a 500-mℓ flask. Into the flask was then charged 200 mℓ of toluene. The reaction system was then heated under reflux so that water was azeotropically boiled away. To the reaction system were then added 100 mℓ of methanol and 3 mℓ of concentrated sulfuric acid. The reaction system was then heated under reflux in a stream of nitrogen for 2 hours. After the completion of the reaction, the reaction solution was poured into 1 ℓ of distilled water, and then extracted with toluene. The resulting organic phase was thoroughly washed with dilute aqueous solution of sodium carbonate and distilled water, and then dried over anhydrous sodium sulfate. The solvent was then distilled off under reduced pressure. The residue was then recrystallized from methanol to obtain 13.4 g of N-(3,4-dimethylphenyl)-N-[4-(3-methoxycarbonylpropyl)phenyl]amine.

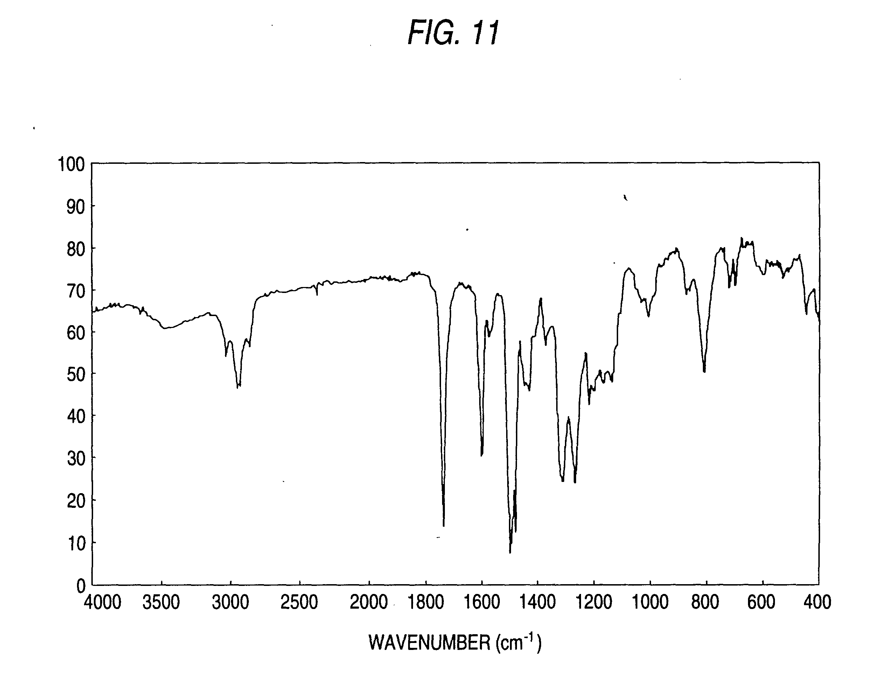

Into a 500-mℓ flask were charged 6.7 g of N-(3,4-dimethylphenyl)-N-[4-(3-methoxycarbonylpropyl)phenyl]amine thus obtained, 3.9 g of 4,4'-diiodo-3,3'-dimethylbiphenyl, 3.8 g of potassium carbonate, 0.5 g of copper sulfate pentahydrate, and 10 mℓ of n-tridecane. The reaction mixture was then allowed to undergo reaction at an elevated temperature of 230°C in a stream of nitrogen for 10 hours. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene to obtain 6.5 g of Compound (III-24) in the form of oily matter. IR spectrum of Compound (III-24) thus obtained is shown in Fig. 11.

### EXAMPLE 12 (Synthesis of III-42)

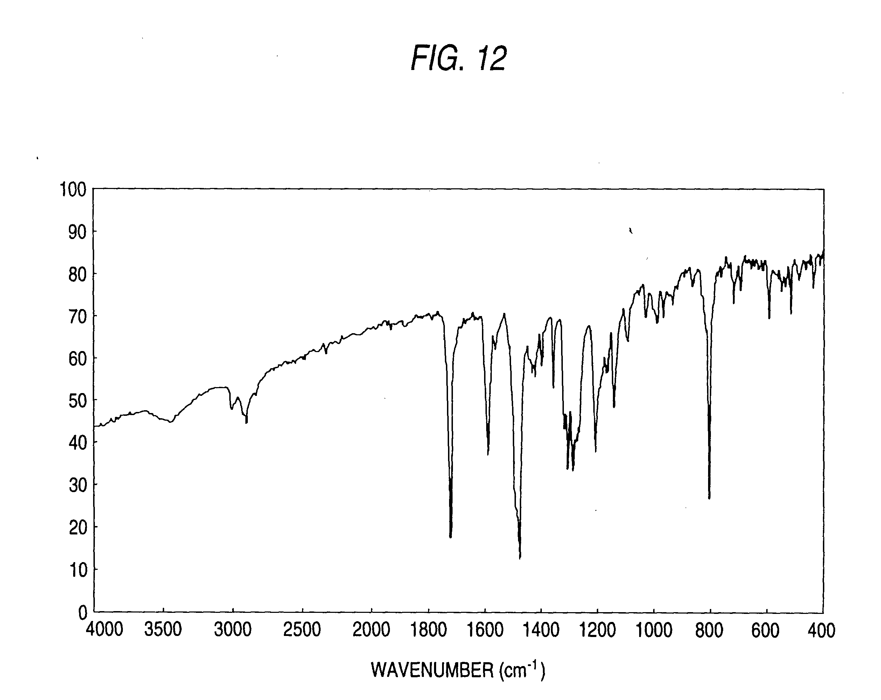

Into a 200-mℓ flask were charged 5.0 g of N-(3,4-dimethylphenyl)-N-[4-(2-methoxycarbonylethyl)phenyl]amine obtained in Example 8, 3.8 g of 4,4'-diiodo-3,3'-dimethylbiphenyl, 2.9 g of potassium carbonate, 1.0 g of copper sulfate pentahydrate, and 10 mℓ of n-tridecane. The reaction mixture was then allowed to undergo reaction at an elevated temperature of 230°C in a stream of nitrogen for 5 hours. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene. The material was then recrystallized from acetone to obtain 3.7 g of Compound (III-42) in the form of light yellow powder. The melting point of Compound (III-24) thus obtained was from 146°C to 147°C. IR spectrum of the compound thus obtained is shown in Fig. 12.

### REFERENCE SYNTHESIS EXAMPLE 13 (Synthesis of (III-53))

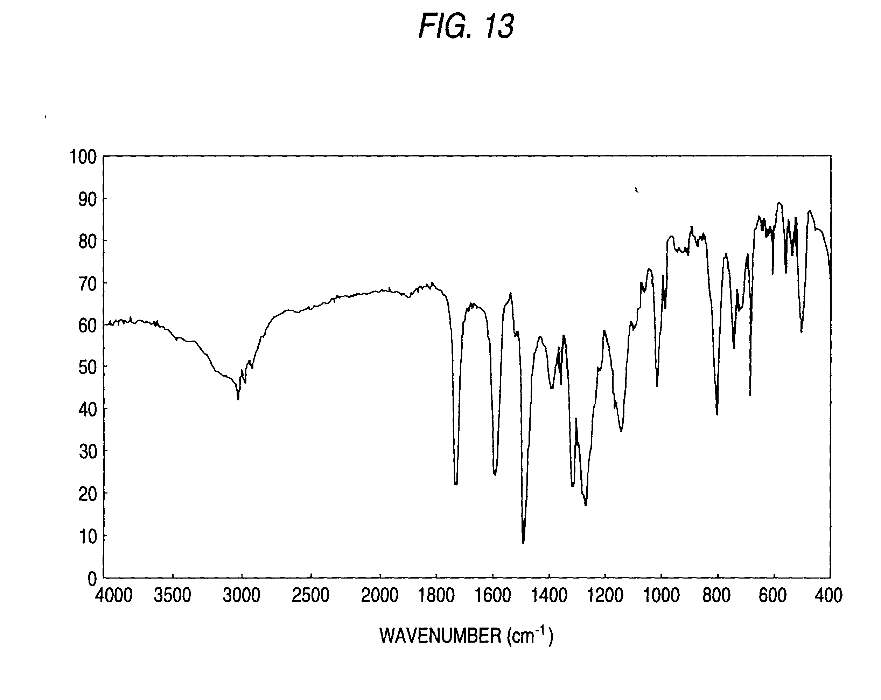

Into a 500-mℓ flask were charged 15.0 g of N, N'-diphenylbenzidine, 35.0 g of Compound (I-30), 16 g of potassium carbonate, 1.0 g of copper sulfate pentahydrate, and 30 mℓ of n-tridecane. The reaction mixture was then allowed to undergo reaction at an elevated temperature of 230°C in a stream of nitrogen for 1 hour. After the completion of the reaction, the reaction system was allowed to cool to room temperature. The reaction system was then dissolved in 100 mℓ of toluene. The insoluble matters were then filtered off. The filtrate was then purified by silica gel column chromatography with toluene to obtain 27.0 g of Compound (III-53) in the form of oily matter. IR spectrum of Compound (III-53) thus obtained is shown in Fig. 13.

### REFERENCE EXAMPLE 14 (Synthesis of III-55)

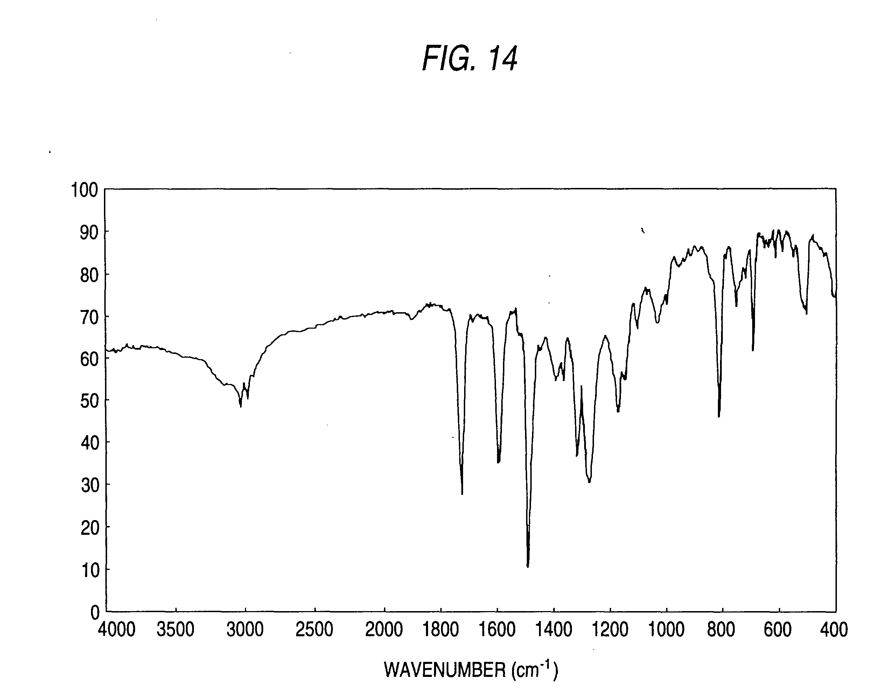

Into a 200-mℓ flask were charged 10.0 g of N, N'-diphenylbenzidine, 24.0 g of Compound (I-34), 11.0 g of potassium carbonate, 1.0 g of copper sulfate pentahydrate, and 30 mℓ of n-tridecane. The reaction mixture was then allowed to undergo reaction at an elevated temperature of 230°C in a stream of nitrogen for 1 hour. After the completion of the reaction, the reaction system was allowed to cool to room temperature. The reaction system was then dissolved in 10 mℓ of toluene. The insoluble matters were then filtered off. The filtrate was then purified by silica gel column chromatography with toluene to obtain 16.6 g of Compound (III-55) in the form of oily matter. IR spectrum of Compound (III-55) thus obtained is shown in Fig. 14.

### EXAMPLE 15 (Synthesis of IV-12)

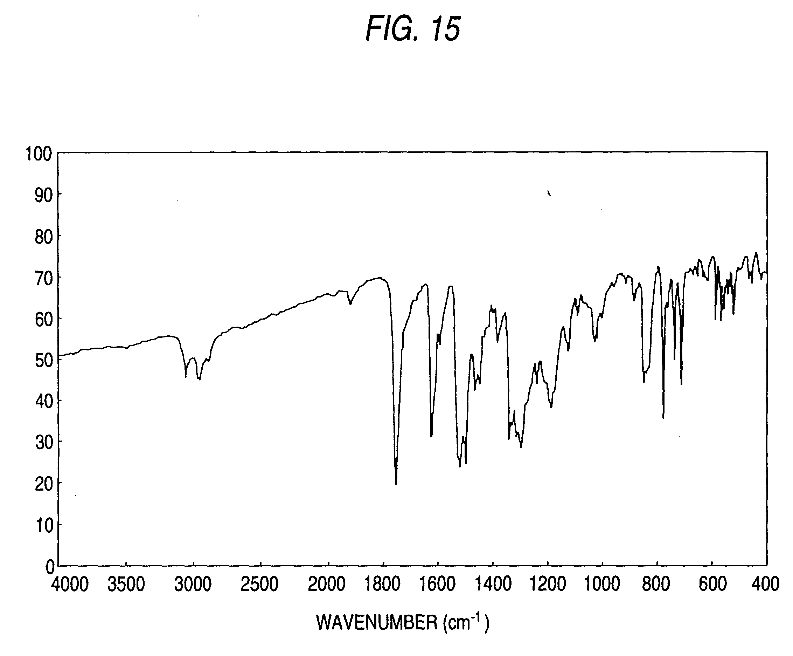

Into a 100-mℓ flask were charged 5.0 g of N-(3,4-dimethylphenyl)-N-[4-(2-methoxycarbonylethyl)phenyl]amine obtained in the same manner as in Example 8, 4.8 g of 4-iodobiphenyl, 2.8 g of potassium carbonate, 0.5 g of copper sulfate pentahydrate, and 5 mℓ of n-tridecane. The reaction mixture was then allowed to undergo reaction at an elevated temperature of 230°C in a stream of nitrogen for 5 hours. After the completion of the reaction, the reaction system was allowed to cool to room temperature. The reaction system was then dissolved in 20 mℓ of toluene. The insoluble matters were then filtered off. The filtrate was then purified by silica gel column chromatography with toluene. The material was then recrystallized from a mixture of ethyl acetate and ethanol to obtain 4.5 g of Compound (IV-12) in the form of light yellow powder. The melting point of Compound (IV-12) thus obtained was from 148°C to 150°C. IR spectrum of the compound thus obtained is shown in Fig. 15.

### APPLICATION EXAMPLE 1

Into a 200-mℓ flask were charged 10 g of Compound (III-7) obtained in Reference Example 4, 20 g of ethylene glycol, and 0.1 g of titanium tetrabutoxide. The reaction mixture was then heated under reflux in a stream of nitrogen for 3 hours. After it was confirmed that Compound (III-7) had been consumed by the reaction, the pressure in the reaction system was reduced to 0.5 mmHg where it was then heated to a temperature of 230°C while distilling off ethylene glycol. The reaction lasted for 3 hours. Thereafter, the reaction system was allowed to cool to room temperature, and then dissolved in 100 mℓ of methylene chloride. Insoluble matters were filtered off. The filtrate was then added dropwise to 1,000 mℓ of acetone with stirring to cause the precipitation of a polymer.

The polymer thus obtained was dissolved in 100 mℓ of tetrahydrofuran (THF), and then filtered. The filtrate was then added dropwise to 1,000 mℓ of water with stirring to cause the precipitation of a polymer. The polymer was thoroughly washed with water, and then dried to obtain 8.4 g of a polymer. The molecular weight Mw of the polymer thus obtained was 1.10 x 10⁵ as determined by GPC in styrene equivalence. (Polymerization degree p: approx. 165)

### APPLICATION EXAMPLE 2

Into a 500-mℓ flask were charged 20 g of Compound (III-18) obtained in Example 8, 40 g of ethylene glycol, and 0.1 g of titanium tetrabutoxide. The reaction mixture was then heated under reflux in a stream of nitrogen for 3 hours. After it was confirmed that Compound (III-18) had been consumed by the reaction, the pressure in the reaction system was reduced to 0.5 mmHg where it was then heated to a temperature of 230°C while distilling off ethylene glycol. The reaction lasted for 3 hours. Thereafter, the reaction system was allowed to cool to room temperature, and then dissolved in 200 mℓ of methylene chloride. Insoluble matters were filtered off. The filtrate was then added dropwise to 1,500 mℓ of ethanol with stirring to cause the precipitation of a polymer. The polymer thus obtained was withdrawn by filtration, thoroughly washed with ethanol, and then dried to obtain 19.2 g of a polymer. The molecular weight Mw of the polymer thus obtained was 1.21 x 10⁵ as determined by GPC in styrene equivalence. (Polymerization degree p: approx. 165)

### APPLICATION EXAMPLE 3

A solution of 100 parts of a zirconium compound (trade name: Orgatics ZC540, available from Matsumoto Seiyaku K.K.), 10 parts of a silane compound (trade name: A1110, available from Nippon Unicar Company Limited), 400 parts of i-propanol and 200 parts of butanol was applied to a honed cylindrical aluminum substrate having a diameter of 30 mmφ by a dip coating method, and then dried at an elevated temperature of 150°C for 10 minutes to form an undercoat layer having a thickness of 0.5 µm.

Subsequently, to the undercoat layer was applied by a dip coating method a coating solution obtained by a process which comprises mixing 10 parts of a chlorogallium phthalocyanine pigment having strong diffraction peaks at 2θ±0.2°=7.4°, 16.6°, 25.5° and 28.3° in powder X-ray diffraction spectrum with 10 parts of a polyvinyl butyral resin (trade name: S-lec BM-S, available from Sekisui Chemical Co., Ltd.) and 500 parts of n-butyl acetate, and then subjecting the mixture to dispersion with glass beads by means of a paint shaker for 1 hour. The material was then dried at a temperature of 100°C for 10 minutes.

Subsequently, to the cylindrical aluminum substrate on which an electric charge-generating layer had been formed was applied by a dip coating method a coating solution obtained by dissolving 5 parts of the electric charge-transporting polymer synthesized in Application Example 1 in 38 parts of monochlorobenzene. The material was then dried at a temperature of 120°C for 1 hour to form an electric charge-transporting layer having a thickness of 15 µm.

### APPLICATION EXAMPLE 4

The procedure of Application Example 3 was followed to prepare an electrophotographic photoreceptor except that 5 parts of the electric charge-transporting polymer synthesized in Application Example 2 were used instead of 5 parts of the electric charge-transporting polymer synthesized in Application Example 1.

### APPLICATION EXAMPLE 5

The procedure of Application Example 3 was followed to prepare an electrophotographic photoreceptor except that 2.5 parts of Compound (III-6) synthesized in Application Example 3 and 2.5 parts of a polycarbonate resin (PC(Z)) were used instead of 5 parts of the electric charge-transporting polymer synthesized in Application Example 1.

### APPLICATION EXAMPLE 6

The procedure of Application Example 3 was followed to prepare an electrophotographic photoreceptor except that 2.5 parts of Compound (III-14) synthesized in Application Example 6 and 2.5 parts of a polycarbonate resin (PC(Z)) were used instead of 5 parts of the electric charge-transporting polymer synthesized in Application Example 1.

The electrophotographic photoreceptors obtained in Application Examples 3 to 6 were evaluated for electrophotographic properties. In some detail, these electrophotographic photoreceptors were each mounted on a Type XP-11 laser beam printer (available from Fuji Xerox Co., Ltd.). With this arrangement, a print test was conducted under ordinary temperature and humidity conditions (20°C, 40%RH). Thus, the image quality after 2,000 sheets of copying was evaluated. The results are set forth in Table 19.

**TABLE 19**

| Application Example No. | Electric charge-transporting layer material | Image quality | |
|---|---|---|---|
| | | 1st sheet | 2,000th sheet |
| 3 | Electric charge-transporting material synthesized in Application Example 1 | Good | Good |
| 4 | Electric charge-transporting material synthesized in Application Example 2 | Good | Good |
| 5 | Compound (III-6)/PC(Z) | Good | Good |
| 6 | Compound (III-14)/PC(Z) | Good | Good |

In the present invention, as can be seen in the foregoing examples, the use of a compound represented by formula (I) can provide an efficient production of amine compounds represented by formulae (II), (III) and (IV).

Further, the amine compounds represented by formulae (II), (III) and (IV) obtained as desired compounds according to the preparation process of the present invention are useful as an electric charge-transporting material or a starting material of electric charge-transporting polymer. Accordingly, the preparation process of the present invention is extremely practical in the preparation of an electrophotographic photoreceptor.

While the invention has been described in detail and with reference to specific examples thereof, it will be apparent to one skilled in the art that various changes and modifications can be made therein without departing from the spirit and scope thereof.

## Claims

1. A process for producing an amine compound represented by formula (II) or formula (III), 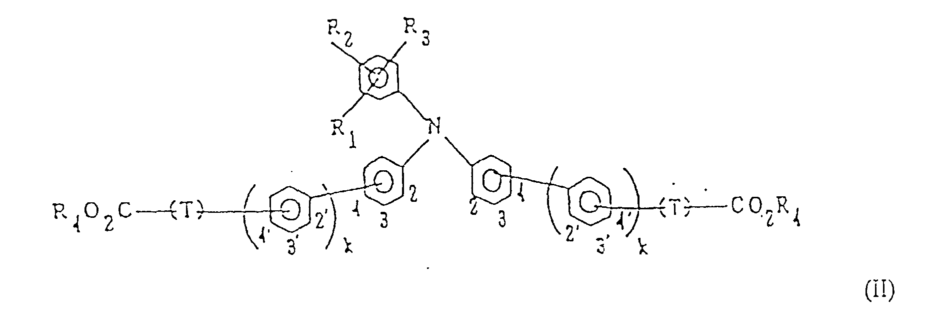 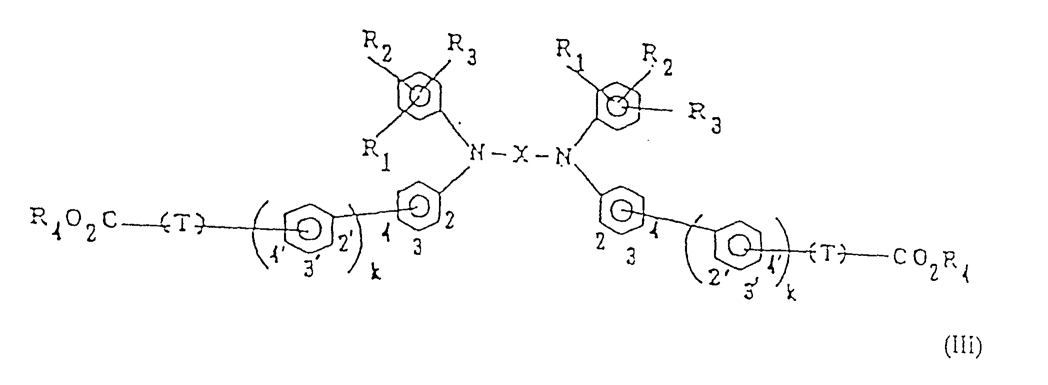 with the exception of N,N'-diphenyl-N,N'-bis[3-(2-ethoxycarbonylethyl)phenyl]-[1,1'-biphenyl]-4,4'-diamine; N,N'-diphenyl-N,N'-bis[4-(4-ethoxycarbonylethylphenyl)phenyl]-[1,1'-biphenyl]-4,4'-diamine, and N,N'-diphenyl-N,N'-bis[4-(4-ethoxycarbonylmethylphenyl)phenyl]-[1,1'-biphenyl]-4,4'-diamine, comprising reacting a compound represented by formula (I) 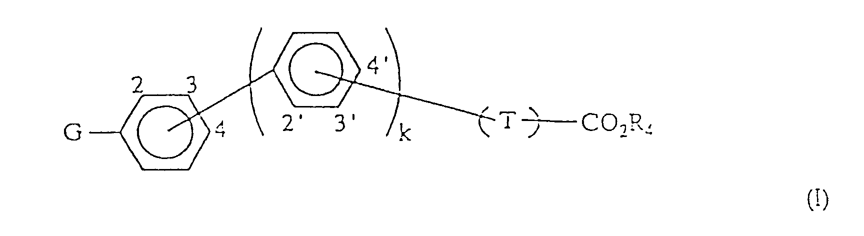 with a compound represented by formula (V) or formula (IX) 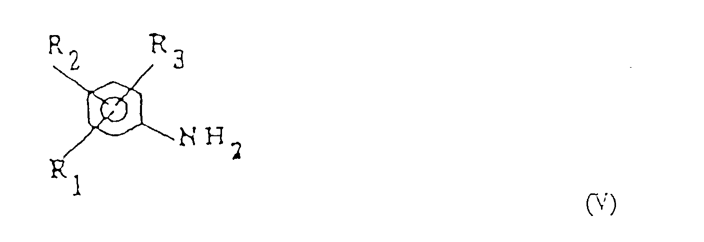 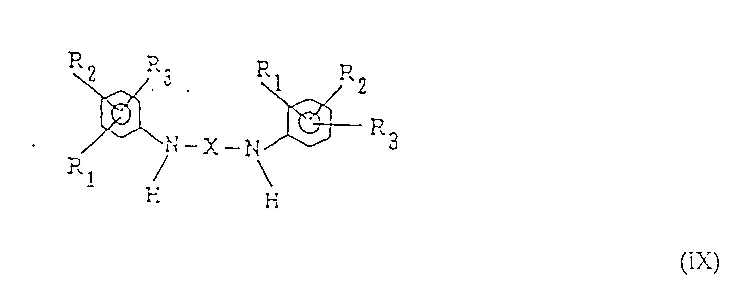 wherein R₁, R₂ or R₃ each independently represents a hydrogen atom, an alkyl group, an alkoxy group, a substituted amino group, a halogen atom or a substituted or unsubstituted aryl group; R₄ represents a hydrogen atom, an alkyl group, a substituted or unsubstituted aryl group or a substituted or unsubstituted aralkyl group; G represents a bromine atom or an iodine atom; X represents a substituted or unsubstituted divalent aryl group; k represents an integer of 0 or 1; and T represents a C₁₋₁₀ divalent hydrocarbon group that may be branched, in the presence of a copper catalyst.

2. The process according to claim 1, wherein the compound represented by formula (I) is coupled with the compound represented by formula (V) to form the compound represented by formula (II).

3. The process according to claim 1, wherein the compound represented by formula (I) is coupled with the compound represented by formula (IX) to form the compound represented by formula (III).

4. The process according to claim 1, wherein said copper catalyst is selected from the group consisting of copper powder, cuprous chloride and copper sulfate.

5. The process according to claim1, wherein 0.001 to 3 parts by weight copper catalyst based on 1 part by weight of the compound of formula (I) is used.

6. The process according to claim 1, wherein said reaction is conducted in the presence of a base.

7. The process according to claim 6, wherein 0.5 to 3 equivalents of the base to the compound of formula (I) is used.

8. The process according to claim 1, wherein said reaction is conducted at a temperature of from 100°C to 300°C.

9. A process for producing an amine compound represented by formula (III) or formula (IV), 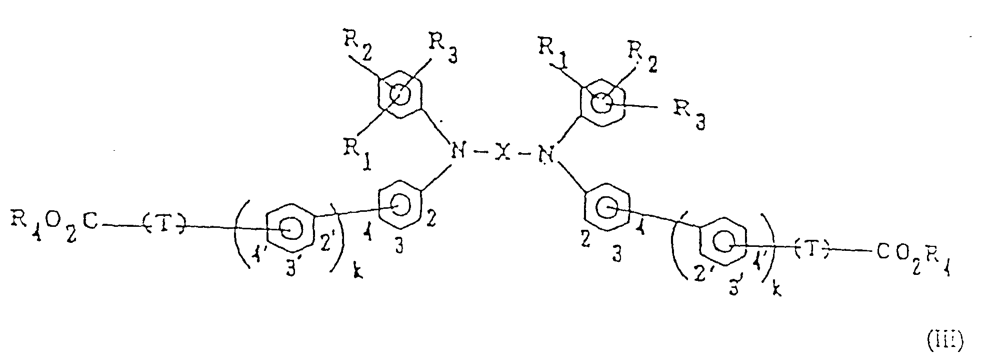 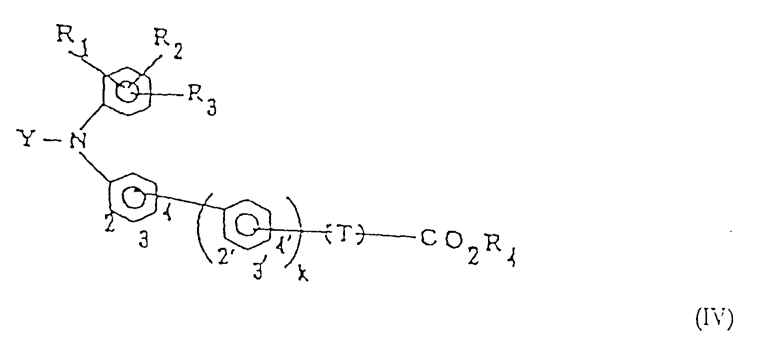 comprising:
1) reacting a compound represented by formula (I) 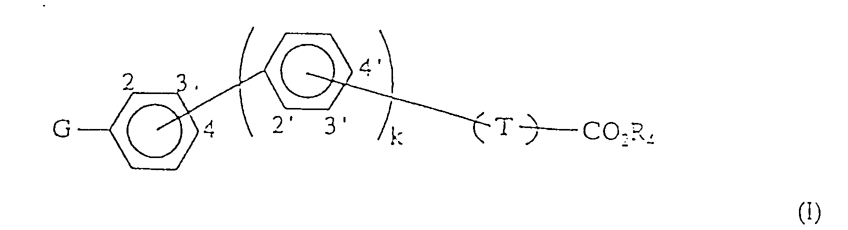 with a compound represented by formula (VI) 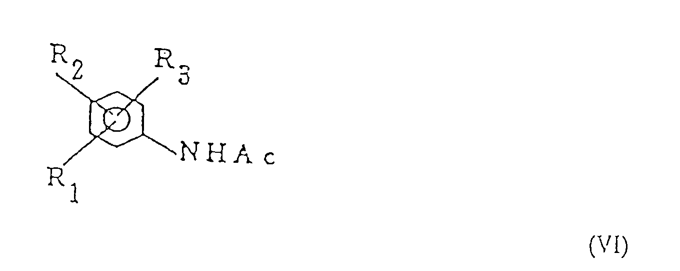 in the presence of a copper catalyst to obtain a reaction product;
2) hydrolyzing the reaction product to obtain a compound represented by formula (VII) or an ester thereof; 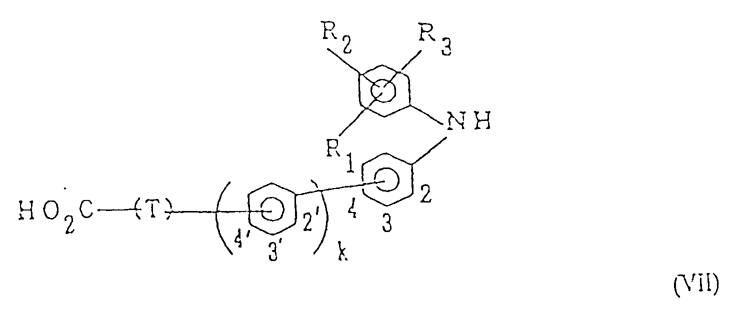 and
3) reacting the compound of formula (VII) or the ester thereof with either a compound of formula (VIII)
G-X-G (VIII)
or
Y-G
wherein R₁, R₂ or R₃ each independently represents a hydrogen atom, an alkyl group, an alkoxy group, a substituted amino group, a halogen atom or a substituted or unsubstituted aryl group; R₄ represents a hydrogen atom, an alkyl group, a substituted or unsubstituted aryl group or a substituted or unsubstituted aralkyl group; G represents a bromine atom or an iodine atom; X represents a substituted or unsubstituted divalent aryl group; k represents an integer of 0 or 1; T represents a C₁₋₁₀ divalent hydrocarbon group that may be branched; and Y represents a substituted or unsubstituted aryl group in the presence of a copper catalyst.

10. The process according to claim 9, wherein the compound represented by formula (VII) or the ester thereof is coupled with the compound represented by formula (VIII) to form the compound represented by formula (III).

11. The process according to claim 9 , wherein the compound represented by formula (VII) or the ester thereof is coupled with Y-G to form the compound represented by formula (IV).

12. The process according to claim 9, wherein the copper catalyst in one or both of steps 1) and 3) is selected from the group consisting of copper powder, cuprous chloride and copper sulfate.

13. The process according to claim 9, wherein 0.001 to 3 parts by weight copper catalyst based on 1 part by weight of the compound of formula (I) is used.

14. The process according to claim 9, wherein at least one step in the reaction is conducted in the presence of a base.

15. The process according to claim 9, wherein in step 1) 0.5 to 3 equivalents of a base to the compound of formula (I) is used.

16. The process according to claim 9, wherein the reaction of step 1) is conducted at a temperature of from 100°C to 300°C.

17. The process according to claim 9, wherein said hydrolyzation reaction is conducted in the presence of a base in a solvent.

18. The process according to claim 17, wherein during said hydrolyzation reaction 0.5 to 10 parts by weight solvent based on 1 part by weight of the compound of formula (I) is used.

19. The process according to claim 17, wherein during said hydrolyzation reaction 0.2 to 5 parts by weight base based on 1 part by weight of the compound of formula (I) is used.

20. The process according to claim 17, wherein said hydrolyzation reaction is conducted at a temperature of from 50°C to the boiling point of the solvent in an atmosphere of inert gas.

## Patentansprüche

1. Verfahren zur Herstellung einer Aminverbindung der Formel (II) oder der Formel (III): 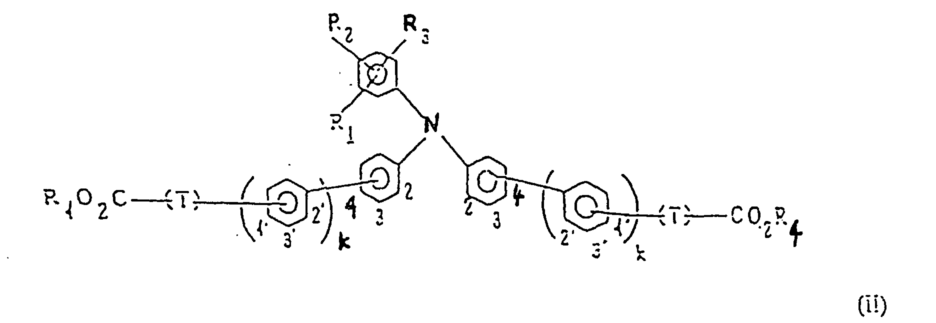 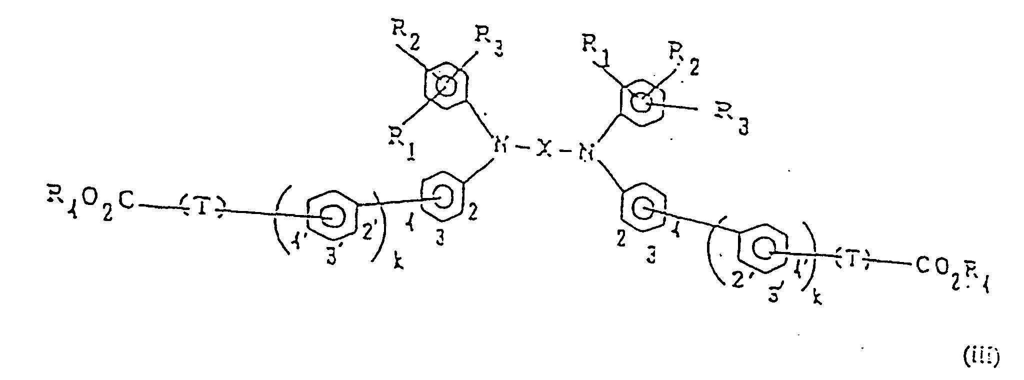 wobei N,N'-Diphenyl-N,N'-bis[3-(2-ethoxycarbonylethyl)phenyl]-[1,1'-biphenyl]-4,4'-diamin, N,N'-Diphenyl-N,N'-bis[4-(4-ethoxycarbonylethylphenyl)phenyl]-[1,1'-biphenyl]-4,4'-diamin und N,N'-Diphenyl-N,N'-bis[4-(4-Ethoxycarbonylmethylphenyl)phenyl]-[1,1'-biphenyl]-4,4'-diamin ausgenommen sind, bei dem eine Verbindung der Formel (I) 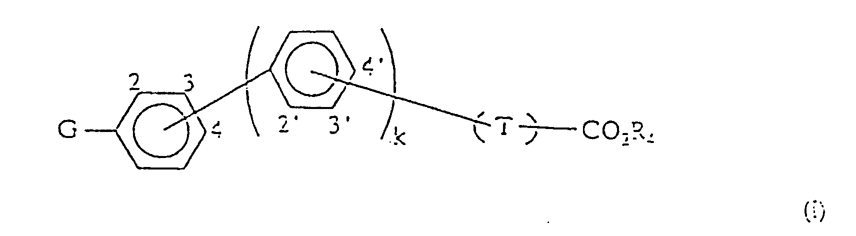 mit einer Verbindung der Formel (V) oder der Formel (IX) 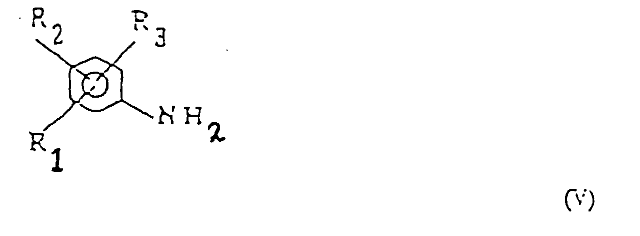 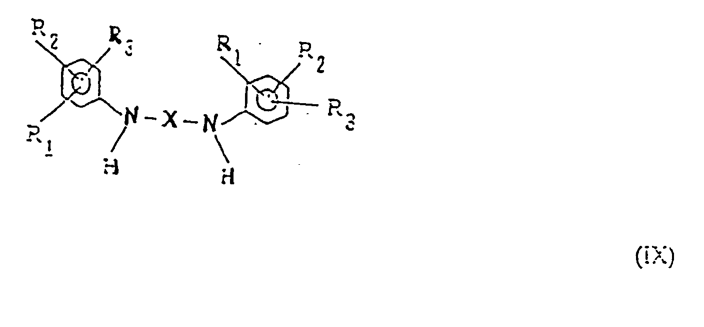 wobei R₁, R₂ oder R₃ jeweils unabhängig voneinander ein Wasserstoffatom, eine Alkyl-Gruppe, eine Alkoxy-Gruppe, eine substituierte Amino-Gruppe, ein Halogenatom oder eine substituierte oder unsubstituierte Aryl-Gruppe bedeuten, R₄ ein Wasserstoffatom, eine Alkyl-Gruppe, eine substituierte oder unsubstituierte Aryl-Gruppe oder eine substituierte oder unsubstituierte Aralkyl-Gruppe bedeutet, G ein Bromatom oder ein Jodatom bedeutet, X eine substituierte oder unsubstituierte zweiwertige Aryl-Gruppe bedeutet, k eine ganze Zahl 0 oder 1 bedeutet und T eine zweiwertige C₁₋₁₀-Kohlenwasserstoffgruppe bedeutet, die verzweigt sein kann, in Gegenwart eines Kupferkatalysators umgesetzt wird.

2. Verfahren nach Anspruch 1, wobei die Verbindung der Formel (I) mit der Verbindung der Formel (V) unter Bildung der Verbindung der Formel (II) gekuppelt wird.

3. Verfahren nach Anspruch 1, wobei die Verbindung der Formel (I) mit der Verbindung der Formel (IX) unter Bildung der Verbindung der Formel (III) gekuppelt wird.

4. Verfahren nach Anspruch 1, wobei der Kupferkatalysator aus der aus Kupferpulver, Kupfer(I)-chlorid und Kupfersulfat bestehenden Gruppe ausgewählt ist.

5. Verfahren nach Anspruch 1, wobei 0,001 bis 3 Gewichtsprozent Kupferkatalysator, bezogen auf 1 Gewichtsteil der Verbindung der Formel (I), verwendet werden.

6. Verfahren nach Anspruch 1, wobei die Umsetzung in Gegenwart einer Base durchgeführt wird.

7. Verfahren nach Anspruch 6, wobei 0,5 bis 3 Äquivalente der Base in bezug auf die Verbindung der Formel (I) verwendet werden.

8. Verfahren nach Anspruch 1, wobei die Umsetzung bei einer Temperatur von 100°C bis 300°C durchgeführt wird.

9. Verfahren zur Herstellung einer Aminverbindung der Formel (III) oder der Formel (IV): 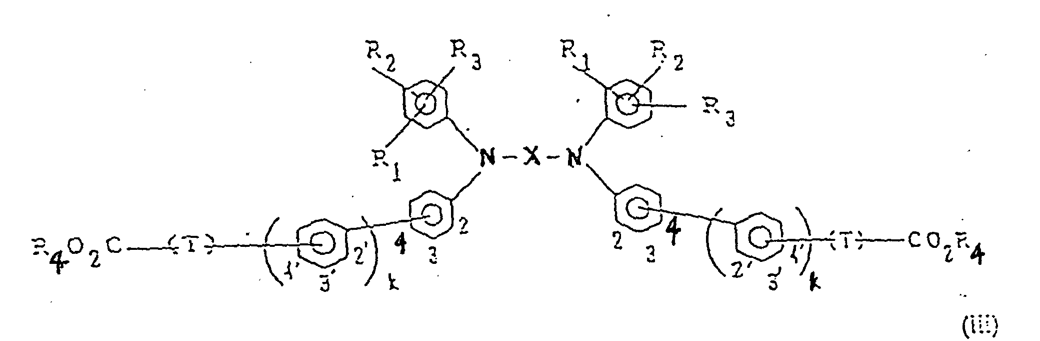 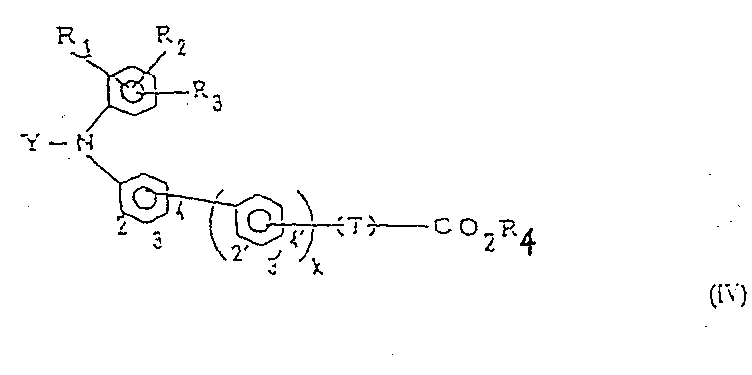 bei dem:
1) eine Verbindung der Formel (I) 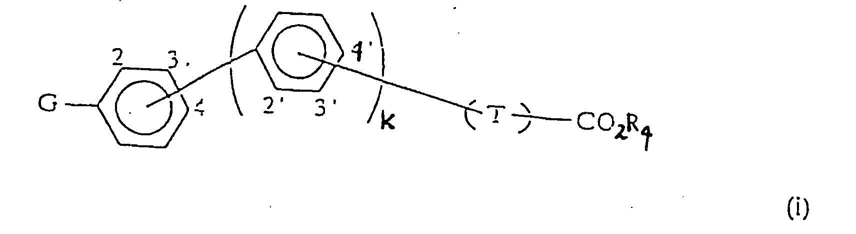 mit einer Verbindung der Formel (VI) 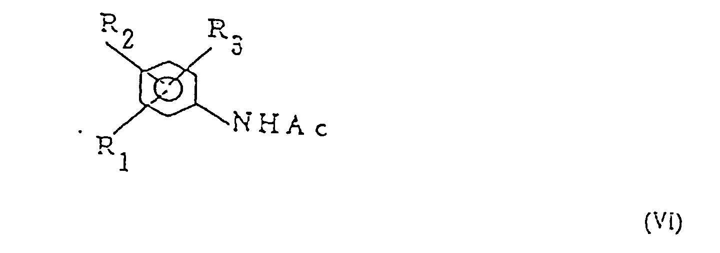 in Gegenwart eines Kupferkatalysators unter Erhalt eines Reaktionsproduktes umgesetzt wird,
2) das Reaktionsprodukt zum Erhalt einer Verbindung der Formel (VII) oder eines Esters davon hydrolysiert wird: 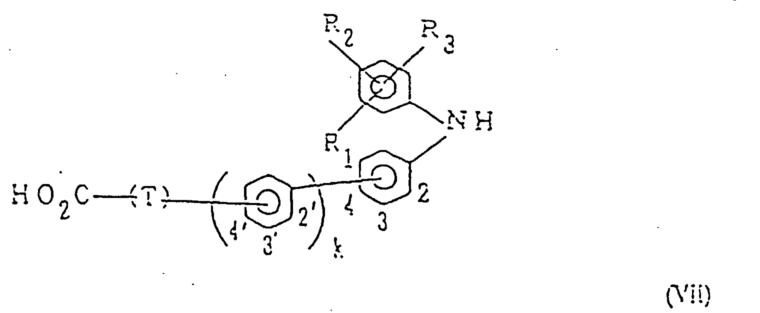 und
3) die Verbindung der Formel (VII) oder deren Ester entweder mit einer Verbindung der Formel (VIII):
G-X-G (VIII)
oder
Y-G,
wobei R₁, R₂ oder R₃ jeweils unabhängig voneinander ein Wasserstoffatom, eine Alkyl-Gruppe, eine Alkoxy-Gruppe, eine substituierte Amino-Gruppe, ein Halogenatom oder eine substituierte oder unsubstituierte Aryl-Gruppe bedeuten, R₄ ein Wasserstoffatom, eine Alkyl-Gruppe, eine substituierte oder unsubstituierte Aryl-Gruppe oder eine substituierte oder unsubstituierte Aralkyl-Gruppe bedeutet, G ein Bromatom oder ein Jodatom bedeutet, X eine substituierte oder unsubstituierte zweiwertige Aryl-Gruppe bedeutet, k eine ganze Zahl 0 oder 1 bedeutet, T eine zweiwertige C₁₋₁₀-Kohlenwasserstoffgruppe bedeutet, die verzweigt sein kann, und Y eine substituierte oder unsubstituierte Aryl-Gruppe bedeutet, in Gegenwart eines Kupferkatalysators umgesetzt wird.

10. Verfahren nach Anspruch 9, wobei die Verbindung der Formel (VII) oder deren Ester mit der Verbindung der Formel (VIII) unter Bildung der Verbindung der Formel (III) gekuppelt wird.

11. Verfahren nach Anspruch 9, wobei die Verbindung der Formel (VII) oder deren Ester mit Y-G unter Bildung der Verbindung der Formel (IV) gekuppelt wird.

12. Verfahren nach Anspruch 9, wobei der Kupferkatalysator in einem oder beiden der Schritte 1) und 3) aus der aus Kupferpulver, Kupfer(I)-chlorid und Kupfersulfat bestehenden Gruppe ausgewählt ist.

13. Verfahren nach Anspruch 9, wobei 0,001 bis 3 Gewichtsteile Kupferkatalysator, bezogen auf 1 Gewichtsteil der Verbindung der Formel (I), verwendet werden.

14. Verfahren nach Anspruch 9, wobei zumindest ein Schritt der Umsetzung in Gegenwart einer Base durchgeführt wird.

15. Verfahren nach Anspruch 9, wobei in Schritt 1) 0,5 bis 3 Äquivalente einer Base, bezogen auf die Verbindung der Formel (I), verwendet werden.

16. Verfahren nach Anspruch 9, wobei der Umsetzungsschritt 1) bei einer Temperatur von 100°C bis 300°C durchgeführt wird.

17. Verfahren nach Anspruch 9, wobei die Hydrolysereaktion in Gegenwart einer Base in einem Lösungsmittel durchgeführt wird.

18. Verfahren nach Anspruch 17, wobei während der Hydrolysereaktion 0,5 bis 10 Gewichtsteile Lösungsmittel, bezogen auf 1 Gewichtsteil der Verbindung der Formel (I), verwendet werden.

19. Verfahren nach Anspruch 17, wobei während der Hydrolysereaktion 0,2 bis 5 Gewichtsteile Base, bezogen auf 1 Gewichtsteil der Verbindung der Formel (I), verwendet werden.

20. Verfahren nach Anspruch 17, wobei die Hydrolysereaktion bei einer Temperatur von 50°C bis zum Siedepunkt des Lösungsmittels in einer Inertgasatmosphäre durchgeführt wird.

## Revendications

1. Procédé de préparation d'un composé d'amine représenté par la formule (II) ou la formule (III), 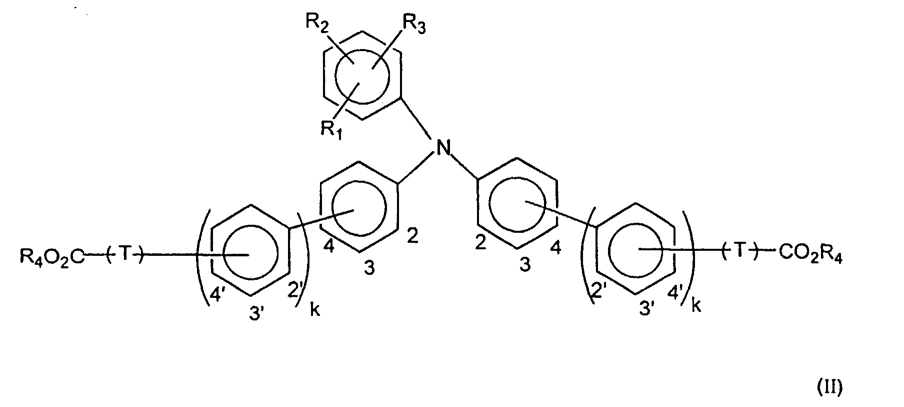 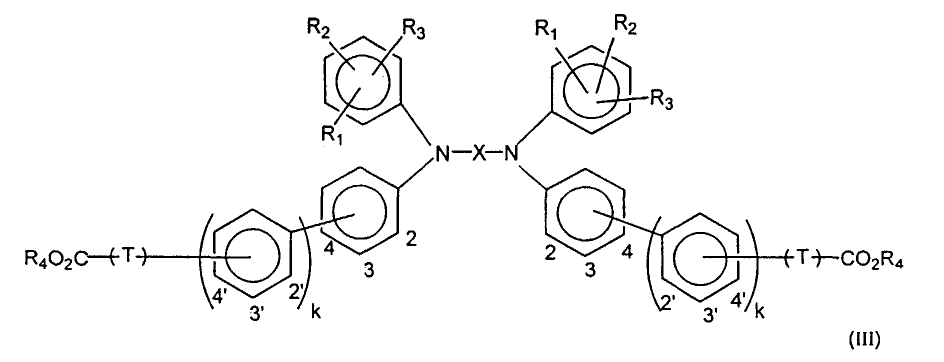 à l'exception de la N,N'-diphényl-N,N'-bis[3-(2-éthoxycarbonyléthyl)-phényl]-[1,1'-bisphényl]-4,4'-diamine; la N,N'-diphényl-N,N'-bis[4-(4-éthoxycarbonyléthylphényl)phényl]-[1,1'-bisphényl]-4,4'-diamine, et la N,N'-diphényl-N,N'-bis[4-(4-éthoxycarbonylméthylphényl)phényl]-[1,1'-bisphényl]-4,4'-diamine, comprenant la réaction d'un composé représenté par la formule (I) 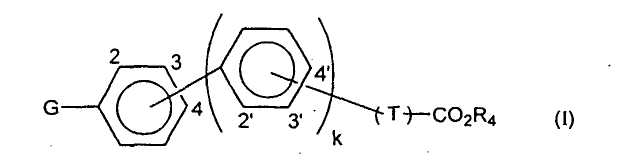 avec un composé représenté par la formule (V) ou (IX) 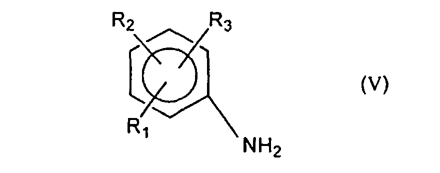 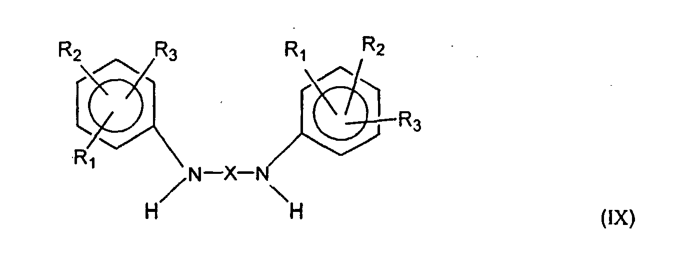 formules dans lesquelles R₁, R₂ ou R₃ représentent chacun indépendamment un atome d'hydrogène, un groupe alkyle, un groupe alcoxy, un groupe amino substitué, un atome d'halogène ou un groupe aryle substitué ou non substitué ; R₄ représente un atome d'hydrogène, un groupe alkyle, un groupe aryle substitué ou non substitué ou un groupe aralkyle substitué ou non substitué ; G représente un atome de brome ou un atome d'iode; X représente un groupe aryle divalent substitué ou non substitué ; k représente un entier égal à 0 ou 1 ; et T représente un groupe hydrocarboné divalent en C₁ à C₁₀ qui peut être ramifié, en présence d'un catalyseur au cuivre.

2. Procédé selon la revendication 1, caractérisé en ce que le composé représenté par la formule (I) est couplé avec le composé représenté par la formule (V) pour former le composé représenté par la formule (II).

3. Procédé selon la revendication 1, caractérisé en ce que le composé représenté par la forrnule (I) est couplé avec le composé représenté par la formule (IX) pour former le composé représenté par la formule (III).

4. Procédé selon la revendication 1, caractérisé en ce que ledit catalyseur au cuivre est choisi dans le groupe constitué par la poudre de cuivre, le chlorure cuivreux et le sulfate de cuivre.

5. Procédé selon la revendication 1, caractérisé en ce qu'on utilise 0,001 à 3 parties en poids de catalyseur au cuivre sur la base d'1 partie en poids du composé de formule (I).

6. Procédé selon la revendication 1, caractérisé en ce qu'on effectue ladite réaction en présence d'une base.

7. Procédé selon la revendication 6, caractérisé en ce qu'on utilise 0,5 à 3 équivalents de la base par rapport au composé de formule (I).

8. Procédé selon la revendication 1, caractérisé en ce qu'on effectue ladite réaction à une température de 100°C à 300°C.

9. Procédé de préparation d'un composé d'amine représenté par la formule (III) ou la formule (IV), 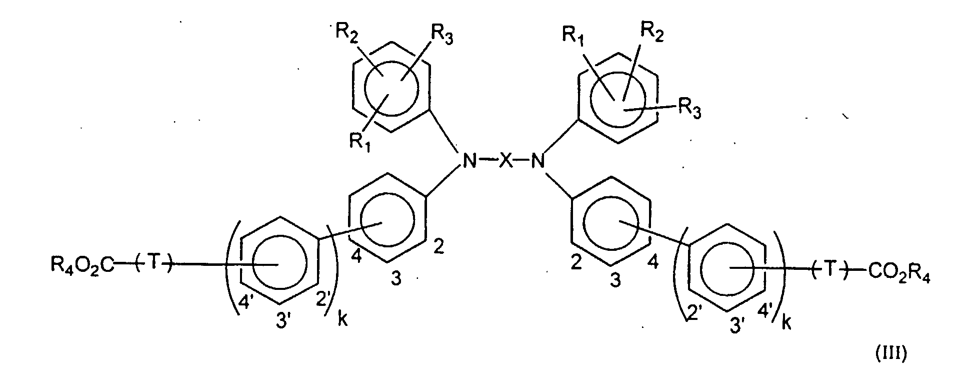 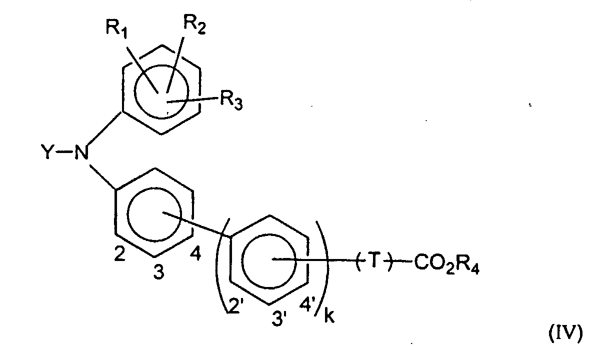 comprenant :
1) la réaction d'un composé représenté par la formule (I) 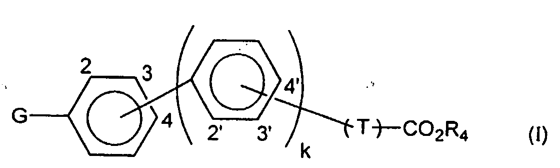 avec un composé représenté par la formule (VI) 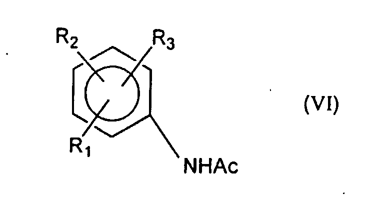 en présence d'un catalyseur au cuivre, pour obtenir un produit réactionnel;
2) l'hydrolyse du produit réactionnel pour obtenir un composé représenté par la formule (VII) ou un ester de celui-ci; 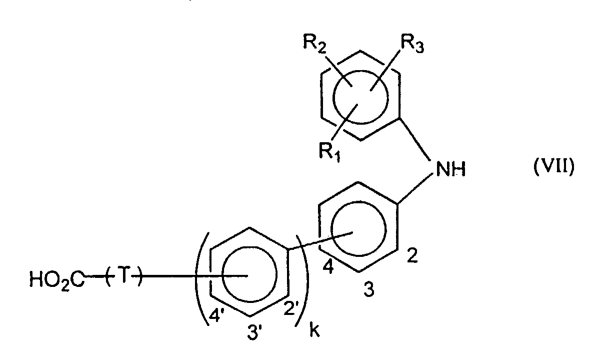 et
3) la réaction du composé de formule (VII) ou l'ester de celui-ci avec un composé de formule (VIII)
G-X-G (VIII)
ou
Y-G
formules dans lesquelles
R₁, R₂ ou R₃ représentent chacun indépendamment un atome d'hydrogène, un groupe alkyle, un groupe alcoxy, un groupe amino substitué, un atome d'halogène ou un groupe aryle substitué ou non substitué; R₄ représente un atome d'hydrogène, un groupe alkyle, un groupe aryle substitué ou non substitué ou un groupe aralkyle substitué ou non substitué ; G représente un atome de brome ou un atome d'iode; X représente un groupe aryle divalent substitué ou non substitué; k représente un entier égal à 0 où 1; et T représente un groupe hydrocarboné divalent en C₁ à C₁₀ qui peut être ramifié ; et Y représente un groupe aryle substitué ou non substitué, en présence d'un catalyseur au cuivre.

10. Procédé selon la revendication 9, caractérisé en ce que le composé représenté par la formule (VII) ou l'ester de celui-ci est couplé avec le composé représenté par la formule (VIII) pour former le composé représenté par la formule (III).

11. Procédé selon la revendication 9, caractérisé en ce que le composé représenté par la formule (VII) ou l'ester de celui-ci est couplé avec Y-G pour former le composé représenté par la formule (IV).

12. Procédé selon la revendication 9, caractérisé en ce que le catalyseur au cuivre de l'une ou des deux étapes 1) et 3) est choisi dans le groupe constitué par la poudre de cuivre, le chlorure cuivreux et le sulfate de cuivre.

13. Procédé selon la revendication 9, caractérisé en ce qu'on utilise 0,001 à 3 parties en poids de catalyseur au cuivre sur la base d'1 partie en poids du composé de formule (I).

14. Procédé selon la revendication 9, caractérisé en ce qu'on effectue au moins une étape dans la réaction en présence d'une base.

15. Procédé selon la revendication 9, caractérisé en ce qu'on utilise dans l'étape 1) 0,5 à 3 équivalents de la base par rapport au composé de formule (I).

16. Procédé selon la revendication 9, caractérisé en ce qu'on effectue la réaction de l'étape 1) à une température de 100°C à 300°C.

17. Procédé selon la revendication 9, caractérisé en ce qu'on effectue ladite réaction d'hydrolyse en présence d'une base dans un solvant.

18. Procédé selon la revendication 17, caractérisé en ce qu'on utilise pendant ladite réaction d'hydrolyse 0,5 à 10 parties en poids de solvant sur la base d'1 partie en poids du composé de formule (I).

19. Procédé selon la revendication 17, caractérisé en ce qu'on utilise pendant ladite réaction d'hydrolyse 0,2 à 5 parties en poids de base sur la base d'1 partie en poids du composé de formule (I).

20. Procédé selon la revendication 17, caractérisé en ce qu'on effectue ladite réaction d'hydrolyse à une température de 50°C au point d'ébullition du solvant dans une atmosphère d'un gaz inerte.
